# EUROPÄISCHE PATENTSCHRIFT

(11) **EP 1 824 338 B1**
(45) Veröffentlichungstag und Bekanntmachung des Hinweises auf die Patenterteilung: **19.03.2008**
(21) Anmeldenummer: 05807937.7
(22) Anmeldetag: 10.11.2005
(51) Int. Cl.: A01N 43/56, C07D 231/20, C07D 231/18, C07D 403/12, C07D 405/12

(54) **3-CYCLOPROPYL-4-(3-AMINO-2-METHYLBENZOYL)PYRAZOLE UND IHRE VERWENDUNG ALS HERBIZIDE**
3-CYCLOPROPYL-4-(3-AMINO-2-METHYLBENZOYL)PYRAZOLS AND THE USE OF THE SAME AS HERBICIDES
3-CYCLOPROPYL-4-(3-AMINO-2-METHYLBENZOYL)PYRAZOLES ET LEUR UTILISATION COMME HERBICIDES

(30) Priorität: 09.12.2004 DE 102004059302
(43) Veröffentlichungstag der Anmeldung: 29.08.2007
(73) Patentinhaber: Bayer CropScience AG, 40789 Monheim (DE)
(72) Erfinder: SCHMITT, Monika, 60318 Frankfurt a. M. (DE); WILLMS, Lothar, 65719 Hofheim (DE); HEINEMANN, Ines, 65719 Hofheim (DE); VAN ALMSICK, Andreas, 61184 Karben (DE); AULER, Thomas, 42799 Leichlingen (DE); HILLS, Martin, 65510 Idstein (DE); KEHNE, Heinz, 65719 Hofheim (DE); FEUCHT, Dieter, 65760 Eschborn (DE)
(86) Internationale Anmeldenummer: PCT/EP2005/012010
(87) Internationale Veröffentlichungsnummer: WO 2006/061074

(56) Entgegenhaltungen:
- WO-A-97/41106
- WO-A-20/05097754
- US-A- 5 824 802
- PATENT ABSTRACTS OF JAPAN Bd. 2000, Nr. 01, 31. Januar 2000 (2000-01-31) -& JP 11 292849 A (NIPPON SODA CO LTD), 26. Oktober 1999 (1999-10-26) in der Anmeldung erwähnt

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus verschiedenen Schriften ist bereits bekannt, dass bestimmte Benzoylpyrazole mit einem Amino-Substituenten in der 3-Position des Benzoyl-Rings herbizide Eigenschaften besitzen. So sind in JP 11 292849 3-Alkyl-4-(3-aminobenzoyl)-pyrazole beschrieben, deren Aminogruppe durch verschiedene Reste substituiert ist. US 5,824,802 beschreibt ebenfalls Benzoylpyrazole mit einem Amino-Substituenten in der 3-Position des Benzoyl-Rings.

Die aus diesen Schriften bekannten Verbindungen zeigen jedoch häufig eine nicht ausreichende herbizide Wirksamkeit und/oder nicht ausreichende Verträglichkeit gegenüber Kulturpflanzen. Insbesondere zeigen die dort offenbarten Verbindungen keine ausreichende Verträglichkeit gegenüber wichtigen Kulturpflanzen wie Mais, Reis, Getreide und Sojabohne.

Aufgabe der vorliegenden Erfindung ist daher die Bereitstellung von herbizid wirksamen Verbindungen mit - gegenüber den aus dem Stand der Technik bekannten Verbindungen - verbesserten herbiziden Eigenschaften sowie verbesserter Verträglichkeit gegenüber Kulturpflanzen, insbesondere gegenüber Mais, Reis, Getreide und Sojabohne.

Es wurde nun gefunden, dass bestimmte 3-Cyclopropyl-4-(3-amino-2-methylbenzoyl)pyrazole als Herbizide besonders gut geeignet sind. Ein Gegenstand der vorliegenden Erfindung sind daher Verbindungen der Formel (I) oder deren Salze 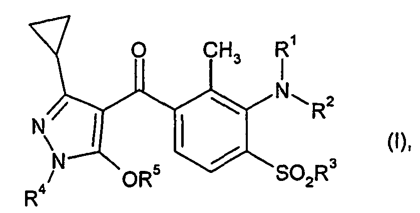 worin
R¹ und R² bedeuten unabhängig voneinander Wasserstoff, Furan-2-yl, Tetrahydrofuran-2-yl-methyl,
   oder durch m Reste aus der Gruppe Fluor, Chlor, Brom, Cyano, Hydroxy, (C₁-C₄)-Alkyl, (C₁-C₄)-Alkoxy und (C₁-C₄)-Alkoxy-(C₁-C₄)-alkoxy substituiertes (C₁-C₄)-Alkyl, (C₃-C₄)-Alkenyl, (C₃-C₄)-Alkinyl, (C₃-C₆)-Cycloalkyl, (C₃-C₆)-Cycloalkenyl, (C₃-C₆)-Cycloalkyl-(C₁-C₄)-alkyl oder (C₃-C₆)-Cycloalkenyl-(C₁-C₄)-alkyl, wobei R¹ und R² nicht beide gleichzeitig Wasserstoff bedeuten,
   oder
   NR¹R² bedeutet einen 4- bis 7-gliedrigen gesättigten, teilweise gesättigten, vollständig ungesättigten oder aromatischen Ring enthaltend als Ringatome n Heteroatome aus der Gruppe Stickstoff, Sauerstoff und Schwefel, der durch m Reste aus der Gruppe Fluor, Chlor, Brom, Iod, Cyano, Hydroxy, Trifluormethyl, Nitro, (C₁-C₄)-Alkyl, (C₁-C₄)-Alkoxy, Fluor-(C₁-C₃)-alkyl, Fluor-(C₁-C₃)-alkoxy oder (C₁-C₃)-Alkoxymethyl substituiert ist;
R³ bedeutet Methyl, Ethyl oder iso-Propyl;
R⁴ bedeutet (C₁-C₄)-Alkyl, (C₃-C₄)-Alkenyl oder (C₃-C₄)-Alkinyl;
R⁵ bedeutet Wasserstoff, (C₁-C₄)-Alkylsulfonyl, (C₃-C₄)-Alkenylsulfonyl, (C₃-C₄)-Alkinylsulfonyl,
   oder durch m Reste aus der Gruppe Fluor, Chlor, Brom, Cyano, Trifluormethyl, Nitro, Methyl, Ethyl, Methoxy und Ethoxy substituiertes Phenylsulfonyl oder Benzyl,
m bedeutet 0, 1, 2 oder 3;
n bedeutet 1, 2 oder 3.

Für den Fall, dass R⁵ Wasserstoff bedeutet, können die erfindungsgemäßen Verbindungen der Formel (I) in Abhängigkeit von äußeren Bedingungen, wie Lösungsmittel und pH-Wert, in unterschiedlichen tautomeren Strukturen auftreten:

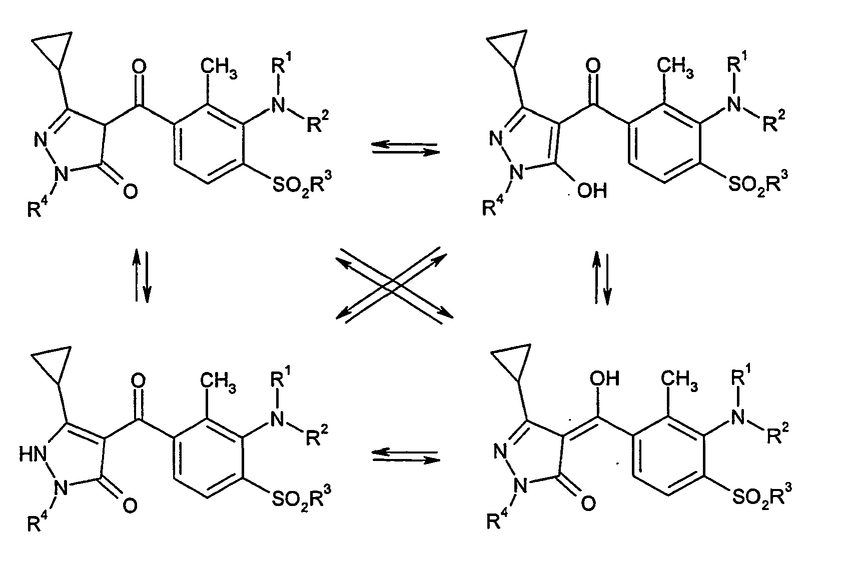

Je nach Art der Substituenten enthalten die Verbindungen der allgemeinen Formel (I) ein acides Proton, das durch Umsetzung mit einer Base entfernt werden kann. Als Basen eignen sich beispielsweise Hydride, Hydroxide und Carbonate von Lithium, Natrium, Kalium, Magnesium und Calcium sowie Ammoniak und organische Amine wie Triethylamin und Pyridin. Solche Salze sind ebenfalls Gegenstand der Erfindung.

In Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Halogen steht für Fluor, Chlor, Brom oder Iod. Tosyl steht für 4-Methylphenylsulfonyl.

In ungesättigten Resten wie Alkenyl und Alkinyl kann sich die Mehrfachbindung in beliebiger Position des Restes befinden. So kann beispielsweise der Rest Propinyl für 1-Propinyl oder 2-Propinyl stehen.

Ist eine Gruppe mehrfach durch Reste substituiert, so ist darunter zu verstehen, dass diese Gruppe durch ein oder mehrere gleiche oder verschiedene der genannten Reste substituiert ist.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfaßt, jedoch nicht spezifisch definiert sind.

Der durch die Gruppe NR¹R² gebildete 4- bis 7-gliedrige Ring steht insbesondere für 1-Pyrrolidinyl, 2-Isoxazoldinyl, 2-Isothioazolidinyl, 1-Pyrazolidinyl, 3-Oxazolidinyl, 3-Thiazolidinyl, 1-Imidazolidinyl, 1,2,4-Oxadiazolidin-2-yl, 1,2,4-Oxadiazolidin-4-yl, 1,2,4-Thiadiazolidin-2-yl, 1,2,4-Thiadiazofidin-4-yl, 1,2,4-Triazolidin-1-yl, 1,2,4-Triazolidin-2-yl, 1,3,4-Oxazolidin-3-yl, 1,3,4-Thiadiazolidin-3-yl, 1,3,4-Triazolidin-3-yl, 2,3-Dihydropyrrol-1-yl, 2,5-Dihydropyrrol-1-yl, 2,3-Dihydroisoxazol-2-yl, 2,5-Dihydroisoxazol-2-yl, 2,3-Dihydroisothiazol-2-yl, 2,5-Dihydroisothiazol-2-yl, 2,3-Dihydrooxazol-3-yl, 2,3-Dihydrothiazol-3-yl, 2,3-Dihydroimidazol-3-yl, 2,5-Dihydroimidazol-3-yl, 1-Morpholinyl, 1-Piperidinyl, 1-Tetrahydropyridazinyl, 1-Tetrahydropyrimidinyl, 1-Tetrahydropyrazinyl, Tetrahydro-1,3,5-triazin-1-yl, Tetrahydro-1,2,4-triazin-1-yl, Tetrahydro-1,2,4-triazin-2-yl, 1,3-Dihydrooxazin-2-yl, 1-Pyrrolyl, 1-Pyrazolyl, 3-Imidazolyl, 1,2,4-Triazolyl-1-yl, 1,3,4-Triazol-1-yl, 1-Piperidin, 1-Morpholinyl,1-Piperazinyl und 1,4-Diazepane (Homopiperazine), 1,4-Oxazepane (Homomorpholine), 1,4-Thiazepane, 1,2,5-Triazepane, 1,2-Oxazepane, 1-2-Thiazepane, 1,2-Thiazepane-1-oxid, 1,2-Thiazepane-1,1-dioxid.

Von näherem Interesse sind Verbindungen der allgemeinen Formel (I), worin
R¹ und R² bedeuten unabhängig voneinander Wasserstoff,
oder durch m Reste aus der Gruppe Fluor, Chlor, Brom, Cyano, Hydroxy, (C₁-C₄)-Alkyl, (C₁-C₄)-Alkoxy und (C₁-C₄)-Alkoxy-(C₁-C₄)-alkoxy substituiertes (C₁-C₄)-Alkyl, (C₃-C₄)-Alkenyl, (C₃-C₄)-Alkinyl, (C₃-C₆)-Cycloalkyl, (C₃-C₆)-Cycloalkenyl, (C₃-C₆)-Cycloalkyl-(C₁-C₄)-alkyl oder (C₃-C₆)-Cycloalkenyl-(C₁-C₄)-alkyl, wobei R¹ und R² nicht beide gleichzeitig Wasserstoff bedeuten,
oder
NR¹R² bedeutet einen 4- bis 7-gliedrigen gesättigten, teilweise gesättigten, vollständig ungesättigten oder aromatischen Ring enthaltend als Ringatome n Heteroatome aus der Gruppe Stickstoff, Sauerstoff und Schwefel, der durch m Reste aus der Gruppe Fluor, Chlor, Brom, Iod, Cyano, Trifluormethyl, Nitro, (C₁-C₄)-Alkyl, (C₁-C₄)-Alkoxy, Fluor-(C₁-C₃)-alkyl, Fluor-(C₁-C₃)-alkoxy oder (C₁-C₃)-Alkoxymethyl substituiert ist;
und die weiteren Substituenten und Indices die oben genannten Bedeutungen haben.

Bevorzugt sind Verbindungen der allgemeinen Formel (I), worin
R¹ und R² unabhängig voneinander Wasserstoff, Methyl, Butyl, Ethyl, Propyl, Propenyl, Propinyl, Cyclopropyl, Cyclopropylmethyl, Methoxyethyl, Methoxypropyl, 2-Methoxy-1-methylethyl, 2-Ethoxy-1-methylethyl, Hydroxyethyl oder Ethoxyethyl bedeuten,
oder
NR¹R² bilden einen Rest aus der Gruppe 1-Pyrrolyl, 1-Pyrrolidinyl, 1-Pyrazolyl, 1-Piperidin, 1-Morpholinyl und 1-Piperazinyl, der durch m Reste aus der Gruppe Fluor, Chlor, Brom, Iod, Cyano, Trifluormethyl, (C₁-C₄)-Alkyl und (C₁-C₄)-Alkoxy substituiert ist,
und die weiteren Substituenten und Indices die oben genannten Bedeutungen haben.

Besonders bevorzugt sind Verbindungen der allgemeinen Formel (I), worin
R¹ und R² unabhängig voneinander Wasserstoff, Methyl, Butyl, Ethyl, Propyl, Propenyl, Propinyl, Cyclopropyl, Cyclopropylmethyl, Methoxyethyl, Methoxypropyl, 2-Methoxy-1-methylethyl, 2-Ethoxy-1-methylethyl, Hydroxyethyl oder Ethoxyethyl bedeuten,
oder
NR¹R² bilden einen Rest aus der Gruppe 1-Pyrrolyl, 1-Pyrrolidinyl, 1-Pyrazolyl, 1-Piperidin, 1-Morpholinyl und 1-Piperazinyl;
R⁵ Wasserstoff, Propylsulfonyl, Tosyl oder 2,6-Difluorbenzyl bedeutet,
und die weiteren Substituenten und Indices die oben genannten Bedeutungen haben.

Ganz bevorzugt sind Verbindungen der allgemeinen Formel (I), worin
- R³: Methyl oder Ethyl bedeutet;
- R⁴: Methyl oder Ethyl bedeutet;
- R⁵: Wasserstoff bedeutet,
und die weiteren Substituenten und Indices die oben genannten Bedeutungen haben.

In allen nachfolgend genannten Formeln haben die Substituenten und Symbole, sofern nicht anders definiert, dieselbe Bedeutung wie unter Formel (I) beschrieben.

Erfindungsgemäße Verbindungen, in denen R⁵ für Wasserstoff steht, können z.B. nach dem in Schema 1 angegebenen und aus DOS 25 13 750 bekannten Verfahren durch basenkatalysierte Umsetzung eines Benzoesäurehalogenids mit einem Pyrazolon oder gemäß dem in Schema 2 angegebenen und beispielsweise aus EP-A 0 186 117 bekannten Verfahren durch basenkatalysierte Umsetzung eines Benzoesäurehalogenids mit einem Pyrazolon und anschließender Umlagerung in Gegenwart einer Base wie Triethylamin und einer Cyanidquelle wie Acetoncyanhydrin, Trimethylsilylcyanid oder Kaliumcyanid hergestellt werden.

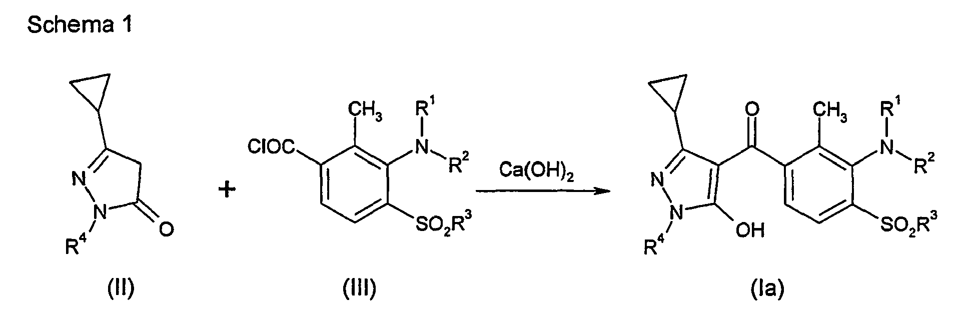

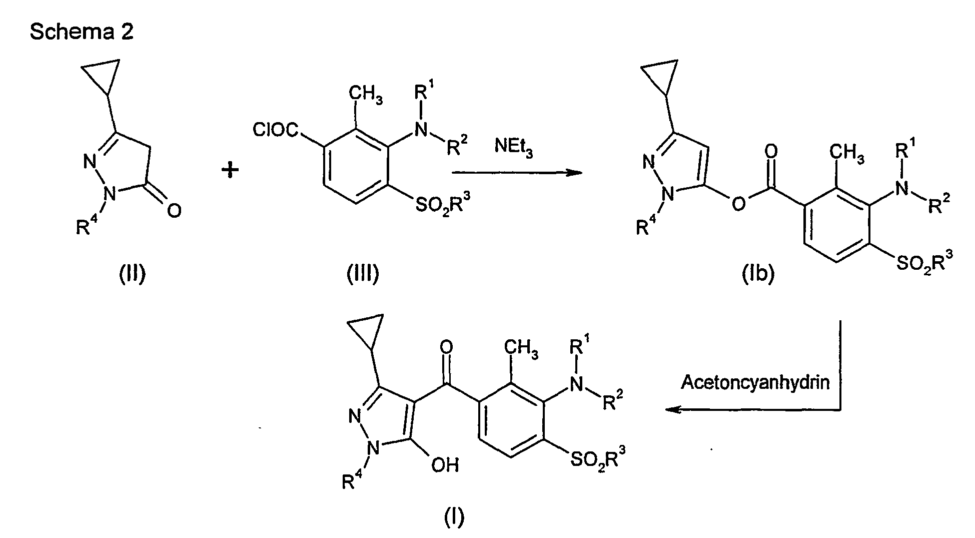

Ebenso können die Verbindungen der allgemeinen Formel (1b) auch direkt aus den entsprechenden Benzoesäuren (IV) in Gegenwart wasserentziehender Reagenzien wie DCC oder EDAC hergestellt werden. Diese Methoden sind dem Fachmann bekannt.

Erfindungsgemäße Verbindungen, in denen R⁵ eine andere Bedeutung als Wasserstoff hat, werden gemäß Schema 3 zweckmäßigerweise aus den nach Schema 1 oder 2 erhältlichen Verbindungen durch basenkatalysierte Reaktion mit einem geeigneten Acylierungsmittel R⁵-X, worin X für eine Abgangsgruppe wie Halogen steht, hergestellt. Solche Methoden sind beispielsweise aus DOS 25 13 750 bekannt.

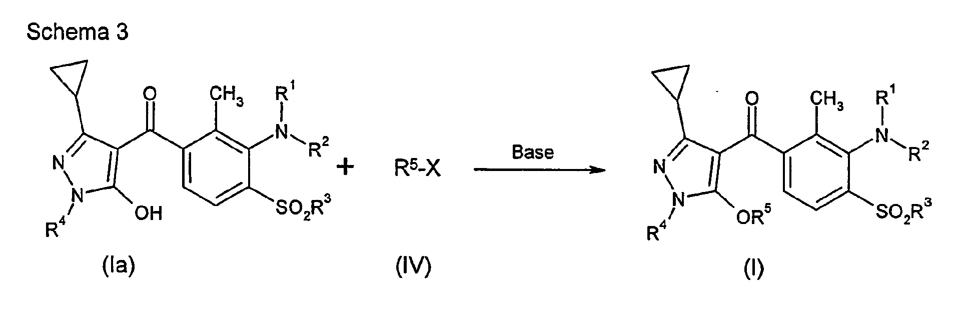

Die in obigen Schemata verwendeten Ausgangsverbindungen sind entweder käuflich oder nach an sich bekannten Methoden herstellbar. Die in obigen Schemata verwendeten Pyrazolone der Formel (II) können nach den aus WO 97/41106 bekannten Methoden hergestellt werden.

Die Benzoesäurehalogenide der allgemeinen Formel (III) können durch ein geeignetes Halogenierungsmittel wie Oxalylchlorid oder Thionylchlorid nach an sich bekannten Methoden aus den entsprechenden Benzoesäuren (IIIa) hergestellt werden.

Die Benzoesäuren der allgemeinen Formel (IIIa) lassen sich beispielsweise aus den entsprechenden 3-Fluor-substituierten Benzoesäuren der allgemeinen Formel (V) herstellen, indem sie unter geeigneten Bedingungen, mit den entsprechenden Aminen HNR¹R² umgesetzt werden (Schema 4). Geeignete Bedingungen sind z.B. das mehrstündige Erhitzen in einem Überschuss des Amins. Solche Reaktionen sind dem Fachmann bekannt.

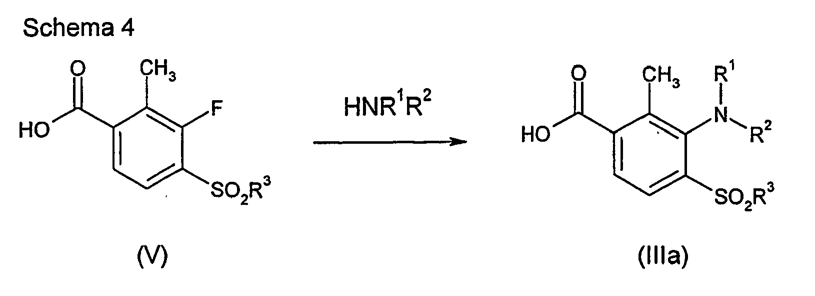

Im Weiteren kann die Gruppe -NR¹R² auch nach ihrer Einführung z.B. durch eine reduktive Aminierung noch derivatisiert werden.

4-Alkylsulfonyl-3-fluor-2-methyl-benzoesäuren der allgemeinen Formel (VI) können gemäß Schema 5 aus 4-Chlor-3-fluor-2-methyl-benzoesäuren (VII) oder deren Ester durch Umsetzung mit Natriumthioalkylaten und anschließender Oxidation mit einem geeigneten Oxidationsmittel erhalten werden. Als Oxidationsmittel eignen sich z.B. Wasserstoffperoxid in Eisessig oder 3-Chlorperbenzoesäure.

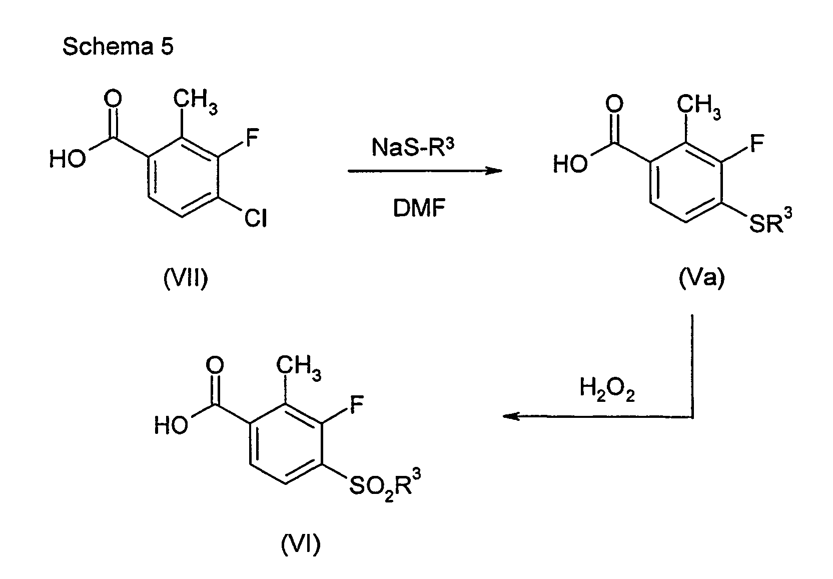

4-Chlor-3-fluor-2-methyl-benzoesäure (VII) ist bekannt und kann z.B. nach der in US 5,334,753 beschriebenen Methode hergestellt werden.

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es in der Regel unerheblich, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll. Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt. Bei dikotylen Unkrautarten erstreckt sich das Wirkungsspektrum auf Arten wie z.B. Galium, Viola, Veronica, Lamium, Stellaria, Amaranthus, Sinapis, Ipomoea, Sida, Matricaria und Abutilon auf der annuellen Seite sowie Convolvulus, Cirsium, Rumex und Artemisia bei den perennierenden Unkräutern. Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Wirkstoffen ebenfalls hervorragend bekämpft. Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird. Insbesondere zeigen die erfindungsgemäßen Verbindungen eine hervorragende Wirkung gegen Apera spica venti, Chenopodium album, Lamium purpureum, Polygonum convulvulus, Stellaria media, Veronica hederifolia, Veronica persica, Viola tricolor sowie gegen Arten von Amaranthus, Galium und Kochia.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Sojabohne nur unwesentlich oder gar nicht geschädigt. Insbesondere weisen sie eine ausgezeichnete Verträglichkeit in Mais, Reis, Getreide und Sojabohne auf. Diese Verbindungen eignen sich daher sehr gut zur selektiven Bekämpfung von unerwünschtem Pflanzenwuchs in landwirtschaftlichen Nutzpflanzungen oder in Zierpflanzungen.

Aufgrund ihrer herbiziden Eigenschaften können diese Verbindungen auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen der Formel (I) oder deren Salze in wirtschaftlich bedeutenden transgenen Kulturen von Nutz- und Zierpflanzen, z. B. von Getreide wie Weizen, Gerste, Roggen, Hafer, Hirse, Reis, Maniok und Mais sowie in Kulturen von Zuckerrübe, Baumwolle, Sojabohne, Raps, Kartoffel, Tomate, Erbse und anderen Gemüsesorten. Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind, insbesondere Sojabohne und Mais.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (z.B. EP-A 0 242 236, EP-A 0 242 246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A 0 142 924, EP-A 0 193 259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431). Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann z.B. erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen. So sind transgene Pflanzen erhältlich, die veränderte Eigenschaften durch Über-expression, Suppression oder Inhibierung homologer (= natürlicher) Gene oder Gensequenzen oder Expression heterologer (= fremder) Gene oder Gensequenzen aufweisen.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen. Gegenstand der Erfindung ist deshalb auch die Verwendung der erfindungsgemäßen Verbindungen als Herbizide zur Bekämpfung von Schadpflanzen in transgenen Kulturpflanzen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Ein weiterer Gegenstand der Erfindung sind deshalb auch herbizide Mittel, die Verbindungen der Formel (I) enthalten. Die Verbindungen der Formel (I) können auf verschiedene Art formuliert werden, je nachdem welche biologischen und/oder chemisch-physikalischen Parameter vorgegeben sind. Als Formulierungsmöglichkeiten kommen z.B. in Frage: Spritzpulver (WP), wasserlösliche Pulver (SP), wasserlösliche Konzentrate, emulgierbare Konzentrate (EC), Emulsionen (EW), wie Öl-in-Wasser- und Wasserin-Öl-Emulsionen, versprühbare Lösungen, Suspensionskonzentrate (SC), Dispersionen auf Öl- oder Wasserbasis, ölmischbare Lösungen, Stäubemittel (DP), Kapselsuspensionen (CS), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, 2,2'-dinaphthylmethan-6,6'-disulfonsaures Natrium, ligninsulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen fein gemahlen und gleichzeitig oder anschließend mit den Formulierungshilfsmittein vermischt.

Emulgierbare Konzentrate werden durch Auflösen des Wirkstoffes in einem organischen Lösungsmittel z.B. Butanol, Cyclohexanon, DMF, Xylol oder auch höhersiedenden Aromaten oder Kohlenwasserstoffen oder Mischungen dieser Lösungsmittel unter Zusatz von einem oder mehreren Tensiden ionischer und/oder nichtionischer Art (Emulgatoren) hergestellt. Als Emulgatoren können z.B., verwendet werden: Alkylarylsulfonsaure Calzium-Salze wie Ca-dodecylbenzol-sulfonat oder nichtionische Emulgatoren wie Fettsäure-polyglykolester, Alkylaryl-polyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylen-sorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde. Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden. Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57. Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I). In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Wirkstoffe in Mischungsformulierungen oder im Tank-Mix sind beispielsweise bekannte Wirkstoffe einsetzbar, wie sie z.B. in Weed Research 26, 441-445 (1986) oder "The Pesticide Manual", 13th edition, The British Crop Protection Council and the Royal Soc. of Chemistry, 2003 und dort zitierter Literatur beschrieben sind. Als bekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und - essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z.B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazapyr; imazamethabenz-methyl; imazaquin und Salze wie das Ammoniumsalz; ioxynil; imazethamethapyr; imazethapyr; imazosulfuron; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; metham; methabenzthiazuron; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monolinuron; monuron; monocarbamide dihydrogensulfate; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; pinoxaden; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl)-oxy]-propansäure und -methylester; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thiazopyr (Mon-13200); thidiazimin (SN-24085); thiobencarb; thifensulfuron-methyl; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha, insbesonders zwischen 0,005 und 250 g/ha.

Die nachstehenden Beispiele erläutern die Erfindung.

### A. Chemische Beispiele

### 1. Herstellung von (3-Cyclopropyl-5-hydroxy-1-methyl-1H-pyrazol-4-yl)[3-[(3-methoxypropyl)amino]-2-methyl-4-(methylsulfonyl)phenyl]methanon (Beispiel 13).

### Schritt 1: 2-Fluor-2-methyl-4-(methylthio)-benzoesäuremethylester

43.3 g (0.214 mol) 4-Chlor-3-fluor-2-methyl-benzoesäuremethylester wurden in 250 ml DMF gelöst und bei Raumtemperatur (RT) mit 17.34 g (0.235 mol) Natriumthiomethylat versetzt. Der Ansatz erwärmte sich auf 65 °C und wurde 4 h bei 50 °C Badtemperatur nachgerührt. Anschließend wurde das Lösungsmittel weitgehend abgezogen, der Rückstand mit 10 %iger H₂SO₄ sauer gestellt und mit EE (Essigsäureethylester) extrahiert. Die organische Phase wurde über MgSO₄ getrocknet und eingeengt. Man erhielt ein gefärbtes Öl einer Reinheit von ca. 60 %.
Rohausbeute: 46 g
¹H-NMR: δ[CDCl₃] 2.47 (s,3H), 2.5 (d,3H), 3.85 (s,3H), 7.0 (t,1H), 7.65 (d,1H)

### Schritt 2: 2-Fluor-2-methyl-4-(methylsulfonyl)-benzoesäuremethylester

40 g roher 2-Fluor-2-methyl-4-(methylthio)-benzoesäuremethylester wurden in 250 ml Eisessig bei 50 °C langsam mit 58 g (0.6 mol) 35 %igem H₂O₂ versetzt. Anschließend wurde 3 h bei 100 °C nachgerührt. Man ließ abkühlen, gab auf 1 I Eiswasser und extrahierte mehrmals mit EE. Die vereinten organischen Phasen wurden über MgSO₄ getrocknet und eingeengt.
Rohausbeute: 46.8 g, ca. 60 % Reinheit (HPLC)
¹H-NMR: δ[CDCl₃] 2.5 (d,3H), 3.21 (s,3H), 3.92 (s,3H), 7.78 (m,2H)

### Schritt 3: 2-Fluor-2-methyl-4-(methylsulfonyl)-benzoesäure

53.8 g roher 2-Fluor-2-methyl-4-(methylsulfonyl)-benzoesäuremethylester wurden in 500 ml THF vorgelegt und mit 10.5 g NaOH gelöst in 500 ml H₂O versetzt. Man ließ 3 h bei RT rühren und zog danach den größten Teil des THF ab. Es wurde mit Diethylether gewaschen und die wässrige Phase anschließend mit 10%iger H₂SO₄ sauer gestellt. Die ausgefallenen Kristalle wurden abgesaugt, mit kaltem Wasser gewaschen und getrocknet. Man erhielt farblose Kristalle.
Rohausbeute: 49.8 g, ca. 70 % Reinheit (HPLC)
¹H-NMR: δ[CDCl₃] 2.52 (d,3H), 3.2 (s,3H), 7.78 (m,2H)

### Schritt 4: 3-[(3-Methoxypropyl)amin]-2-methyl-4-(methylsulfonyl)-benzoesäure

1 g rohe 2-Fluor-2-methyl-4-(methylsulfonyl)-benzoesäure und 11.9 g (133 mmol) 3-Methoxypropylamin wurden vermischt und unter Rühren 24 h bei 120 °C erhitzt. Man ließ abkühlen, gab den Ansatz auf 100 ml Wasser, säuerte mit 10 %iger H₂SO₄ an und extrahierte mehrmals mit EE. Die organischen Phasen wurden über MgSO₄ getrocknet und eingeengt. Man erhielt farblose Kristalle.
Rohausbeute: 1.07 g, ca. 88 % Reinheit (HPLC)
¹H-NMR: δ[CDCl₃] 1.95 (m,2H), 2.52 (d,3H), 3.12 (s,3H), 3.25 (t,2H), 3.39 (s,3H), 3.58 (t,2H), 7.58 (d,2H), 7.8 (d,2H)

### Schritt 5: (3-Cyclopropyl-5-hydroxy-1-methyl-1H-pyrazol-4-yl)[3-[(3-methoxypropyl)amino]-2-methyl-4-(methylsulfonyl)phenyl]methanon

0.53 g rohe 3-[(3-Methoxypropyl)amin]-2-methyl-4-(methylsulfonyl)-benzoesäure wurden unter Stickstoff in 30 ml CH₃CN gelöst und anschließend mit 0.32 g (2 mmol) 5-Hydroxy-3-cyclopropyl-1-methylpyrazol und 0.41 g (2 mmol) EDAC versetzt. Man ließ einen Tag bei RT rühren. Anschließend versetzte man mit 0.36 g (4 mmol) NEt₃, 0.07 g (1 mmol) Me₃SiCN und einer Spatelspitze KCN und ließ zwei Tage bei RT rühren. Danach wurde eingeengt, der Rückstand in 100 ml CH₂Cl₂ aufgenommen und mit 10 %iger H₂SO₄ gewaschen. Die organische Phase wurde über MgSO₄ getrocknet, eingeengt, chromatographisch gereinigt (SiO₂, EE/n-Heptan =1:1 + 5 % Essigsäure) und aus Diethylether kristallisiert. Man erhielt farblose Kristalle.
Ausbeute: 52 mg , (0.12 mmol) 7 %, 96 % Reinheit (HPLC)
¹H-NMR: δ[CDCl₃] 0.53 (m,2H), 0.78 (m,2H), 0.98 (m,1H), 1.95 (m,2H), 2.3 (s,3H), 3.1 (s,3H), 3.28 (t,2H), 3.38 (s,3H), 3.58 (t,2H), 3.6 (s,3H), 6.99 (d,1H), 7.82 (d,1H)

### 2. Herstellung von (3-Cyclopropyl-5-hydroxy-1-methyl-1H-pyrazol-4-yl)[3-[(2-methoxyethyl)(methyl)amino]-2-methyl-4-(methylsulfonyl)phenyl]methanone (Beispiel 24).

### Schritt 1: 3-[(2-Methoxyethyl)(methyl)amino]-2-methyl-4-(methylsulfonyl)-benzoesäure

1.0 g (3 mmol) 3-[(2-Methoxyethyl)amino]-2-methyl-4-(methylsulfonyl)benzoesäure wurden unter Stickstoff in 150 ml THF gelöst, mit 10 g Paraformaldehyd und anschließend portionsweise mit 0,7 g (20 mmol) NaBH₄ versetzt. Danach ließ man langsam 15 ml Trifluoressigsäure zutropfen. Der Ansatz rührte 3 Tage bei RT nach. Zur Aufarbeitung wurde mit 10 %iger H₂SO₄ versetzt, mehrmals mit EE extrahiert und dann über MgSO₄ getrocknet und eingeengt. Man erhielt ein bräunliches Öl.
Rohausbeute: 1.2 g, 98 % Reinheit (HPLC)
¹H-NMR: δ[CDCl₃] 2.52 (d,3H), 2.92 (s,3H), 3.35 (s,6H), 3.42 (m,2H), 3.65 (m,2H), 7.8 (d,2H), 7.98 (d,2H)

### Schritt 2: (3-Cyclopropyl-5-hydroxy-1-methyl-1H-pyrazol-4-yl)[3-[(2-methoxyethyl)(methyl)-amino]-2-methyl-4-(methylsulfonyl)phenyl]methanone

0.59 g rohe 3-[(2-Methoxyethyl)amino]-2-methyl-4-(methylsulfonyl)benzoesäure wurden unter Stickstoff in 30 ml CH₃CN gelöst und anschließend mit 0.35 g (3 mmol) 5-Hydroxy-3-cyclopropyl-1-methylpyrazol und 0.45 g (2 mmol) EDAC versetzt. Man ließ einen Tag bei RT rühren. Anschließend versetze man mit 0.36 g (4 mmol) NEt₃, 0.08 g (1 mmol) Me₃SiCN und einer Spatelspitze KCN und ließ zwei Tage bei RT rühren. Danach wurde eingeengt, der Rückstand in 100 ml CH₂Cl₂ aufgenommen und mit 10 %iger H₂SO₄ gewaschen. Die organische Phase wurde über MgSO₄ getrocknet, eingeengt und der Rückstand chromatographisch gereinigt (SiO₂, Essigsäureethylester/n-Heptan = 1:1 + 5 % Essigsäure). Man erhielt ein braunes Öl.
Ausbeute: 144 mg , (0.34 mmol) 16 %, 93 % Reinheit (HPLC)
¹H-NMR: δ[CDCl₃] 0.53 (m,2H), 0.78 (m,2H), 0.9 (m,1H), 2.35 (s,3H), 2.95 (s,3H), 3.25 (m,2H), 3.17 (s,3H), 3.18 (s,3H), 3.42 (m,2H), 3.6 (s,3H), 3.65 (m,2H), 7.32 (d,1 H), 8.02 (d,1 H)

Die in nachfolgender Tabelle A aufgeführten Beispiele wurden analog obiger Methoden hergestellt beziehungsweise sind analog obiger Methoden erhältlich.

Die verwendeten Abkürzungen bedeuten:
Bu = Butyl Et = Ethyl Me = Methyl Pr = Propyl Ph = Phenyl

**Tabelle A:**

| | | | | | |
|---|---|---|---|---|---|
| 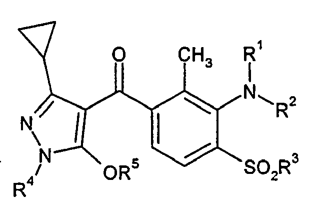 | | | | | |

| **Nr.** | **NR¹R²** | **R³** | **R⁴** | **R⁵** | **¹H-NMR: δ[CDCl₃]** |
|---|---|---|---|---|---|
| 1 | NHMe | Me | Me | H | 0.54 (m,2H), 0.78 (m,2H), 1.01 (m,1 H), 2.33 (s,3H), 3.00 (s,3H), 3.08 (s,3H), 3.62 (s,3H), 7.00 (d,1H), 7.80 (d,1H) |
| 2 | NHEt | Me | Me | H | 0.55 (m,2H), 0.77 (m,2H), 1.00 (m,1H), 1.20 (t,3H), 2.30 (s,3H), 3.07 (s,3H), 3.27 (q,2H), 3.59 (s,3H), 6.98 (d,1 H), 7.80 (d,1 H) |
| 3 | NH(n-Pr) | Me | Me | H | 0.53 (m,2H), 0.78 (m,2H), 0.98 (m,1 H), 1.02 (t,3H), 1.7 (m,2H), 2.3 (s,3H), 3.1 (s,3H), 3.18 (t,2H), 3.6 (s,3H), 6.95 (d,1H), 7.8 (d,1H) |
| 4 | NH(n-Bu) | Me | Me | H | 0.53 (m,2H), 0.78 (m,2H), 0.95 (t,3H), 0.98 (m,1H), 1.45 (m,2H), 1.65 (m,2H), 2.3 (s,3H), 3.1 (s,3H), 3.2 (t,2H), 3.6 (s,3H), 6.95 (d,1 H), 7.8 (d,1 H) |
| 5 | NHCH₂CH=CH₂ | Me | Me | H | 0.53 (m,2H), 0.78 (m,2H), 0.98 (m,1H), 2.3 (s,3H), 3.1 (s,3H), 3.6 (s,3H), 3.85 (d,2H), 5.22 (m,1H), 5.35 (m,1H), 6.0 (m,1H), 7.02 (d,1H), 7.82 (d,1H) |
| 6 | NHCH₂C≡CH | Me | Me | H | |
| 7 | NHCH₂cPr | Me | Me | H | 0.3 (m,2H), 0.53 (m,2H), 0.6 (m,2H), 0.78 (m,2H), 0.98 (m,1H), 1.1 (m,1H), 2.3 (s,3H), 3.08 (d,2H), 3.1 (s,3H), 3.6 (s,3H), 6.95 (d,1 H), 7.81 (d,1 H) |
| 8 | NH(CH₂)₂OH | Me | Me | H | 0.53 (m,2H), 0.78 (m,2H), 0.98 (m,1H), 2.3 (s,3H), 3.2 (s,3H), 3.38 (t,2H), 3.6 (s,3H), 3.85 (t,2H), 7.02 (d, 1H), 7.82 (d, 1H) |
| 9 | NH(CH₂)₂OMe | Me | Me | H | 0.53 (m,2H), 0.78 (m,2H), 0.98 (m, 1 H), 2.3 (s,3H), 3.18 (s,3H), 3.4 (t,2H), 3.4 (s,3H), 3.6 (t,2H), 3.6 (s,3H), 6.98 (d,1H), 7.82 (d,1H) |
| 10 | N NH(CH₂)₂OEt | Me | Me | H | 0.53 (m,2H), 0.78 (m,2H), 0.98 (m,1H), 1.22 (t,3H), 2.3 (s,3H), 3.2 (s,3H), 3.4 (t,2H), 3.58 (q,2H), 3.6 (s,3H), 3.62 (t,2H), 6.95 (d,1H), 7.82 (d,1H) |
| 11 | NHCHMeCH₂OMe | Me | Me | H | 0.53 (m,2H), 0.78 (m,2H), 1.02 (m,1H), 1.22 (d,3H), 2.25 (s,3H), 3.18 (s,3H), 3.3 (s,3H), 3.4 (m,2H), 3.6 (s,3H), 3.82 (m,1H), 6.95 (d,1 H), 7.82 (d,1H) |
| 12 | NHCHMeCH₂OEt | Me | Me | H | |
| 13 | NH(CH₂)₃OMe | Me | Me | H | 0.54 (m,2H), 0.77 (m,2H), 0.99 (m,1 H), 1.94 (m,2H), 2.32 (s,3H), 3.11 (s,3H), 3.29 (t,2H), 3.37 (s,3H), 3.55 (t,2H), 3.60 (s,3H), 6.99 (d,1H), 7.82 (d,1H) |
| 14 | 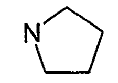 | Me | Me | H | 0.53 (m,2H), 0.78 (m,2H), 0.9 (m,1H), 2.05 (m,4H), 2.3 (s,3H), 3.25 (s,3H), 3.3 (m,4H), 3.6 (s,3H), 7.35 (d,1 H), 8.02 (d,1 H) |
| 15 | 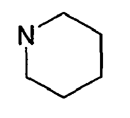 | Me | Me | H | 0.53 (m,2H), 0.78 (m,2H), 0.9 (m, 1H), 1.65-1.95 (m,6H), 2.4 (s,3H), 3.1 (m,2H), 3.3 (m,2H), 3.35 (s,3H), 3.6 (s,3H), 7.3 (d,1H), 8.03 (d,1 H) |
| 16 | NMe₂ | Me | Me | H | 0.50 (m,2H), 0.77 (m,2H), 0.90 (m,1 H), 2.37 (s,3H), 2.93 (s,6H), 3.28 (s,3H), 3.61 (s,3H), 7.33 (d,1 H), 8.02 (d,1 H) |
| 17 | NMeEt | Me | Me | H | 0.51 (m,2H), 0.77 (m,2H), 0.92 (m,1 H), 1.23 (t,3H), 2.34 (s,3H), 2.87 (s,3H), 3.07-3.37 (m,2H), 3.32 (s,3H), 3.60 (s,3H), 7.33 (d,1 H), 8.04 (d,1 H) |
| 18 | NMe(n-Pr) | Me | Me | H | 0.52 (m,2H), 0.78 (m,2H), 0.89 (m,1H), 0.92 (t,3H), 1.67 (m,2H), 2.35 (s,3H), 2.88 (s,3H), 2.93-3.18 (m,2H), 3.30 (s,3H), 3.61 (s,3H), 7.31 (d,1H), 8.03 (d,1H) |
| 19 | NMe(n-Bu) | Me | Me | H | |
| 20 | NMeCH₂CH=CH₂ | Me | Me | H | |
| 21 | NMeCH₂C=CH | Me | Me | H | |
| 22 | NMeCH₂cPr | Me | Me | H | 0.10-0.28 (m,2H), 0.42-0.54 (m,3H), 0.63 (m,1H), 0.76 (m,2H), 0.92 (m,1H), 1.12 (m,1H), 2.33 (s,3H), 2.71 (m,1H), 2.97 (s,3H), 3.28 (m,1 H), 3.36 (s,3H), 3.61 (s,3H), 7.32 (d,1H), 8.04 (d,1H) |
| 23 | NMe(CH₂)₂OH | Me | Me | H | |
| 24 | NMe(CH₂)₂OMe | Me | Me | H | 0.50 (m,2H), 0.77 (m,2H), 0.91 (m,1H), 2.36 (s,3H), 2.94 (s,3H), 3.20-3.28 (m,1H), 3.35 (s,3H), 3.37 (s,3H), 3.39-3.47 (m,2H), 3.61 (s,3H), 3.66 (m,2H), 7.33 (d,1H), 8.03 (d,1H) |
| 25 | NMe(CH₂)₂OEt | Me | Me | H | 0.50 (m,2H), 0.76 (m,2H), 0.90 (m,1H), 1.09 (t,3H), 2.37 (s,3H), 2.95 (s,3H), 3.18-3.28 (m,1H), 3.36 (s,3H), 3.39-3.47 (m,1 H), 3.50 (q,2H), 3.61 (s,3H), 3.70 (m,2H), 7.32 (d,1H), 8.03 (d,1H) |
| 26 | NMeCHMeCH₂OMe | Me | Me | H | |
| 27 | NMeCHMeCH₂OEt | Me | Me | H | |
| 28 | NMe(CH₂)₃OMe | Me | Me | H | 0.52 (m,2H), 0.78 (m,2H), 0.91 (m,1H), 1.96 (m,2H), 2.36 (s,3H), 2.88 (s,3H), 3.04-3.17 (m,1H), 3.23-3.34 (m,1H), 3.27 (s,3H), 3.32 (s,3H), 3.37-3.47 (m,2H), 3.60 (s,3H), 7.32 (d,1 H), 8.03 (d,1H) |
| 29 | NHMe | Et | Me | H | 0.54 (m,2H), 0.78 (m,2H), 0.99 (m,1H), 1.28 (t,3H), 2.33 (s,3H), 2.96 (s,3H), 3.12 (q,2H), 3.61 (s,3H), 6.98 (d,1H), 7.75 (d,1 H) |
| 30 | NHEt | Et | Me | H | |
| 31 | NH(n-Pr) | Et | Me | H | |
| 32 | NH(n-Bu) | Et | Me | H | |
| 33 | NHCH₂CH=CH₂ | Et | Me | H | |
| 34 | NHCH₂C≡CH | Et | Me | H | |
| 35 | NHCH₂cPr | Et | Me | H | |
| 36 | NH(CH₂)₂OH | Et | Me | H | |
| 37 | NH(CH₂)₂OMe | Et | Me | H | |
| 38 | NH(CH₂)₂OEt | Et | Me | H | |
| 39 | NHCHMeCH₂OMe | Et | Me | H | |
| 40 | NHCHMeCH₂OEt | Et | Me | H | |
| 41 | NH(CH₂)₃OMe | Et | Me | H | |
| 42 | 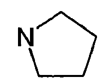 | Et | Me | H | |
| 43 | 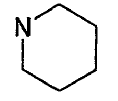 | Et | Me | H | |
| 44 | NMe₂ | Et | Me | H | |
| 45 | NMeEt | Et | Me | H | |
| 46 | NMe(n-Pr) | Et | Me | H | |
| 47 | NMe(n-Bu) | Et | Me | H | |
| 48 | NMeCH₂CH=CH₂ | Et | Me | H | |
| 49 | NMeCH₂C≡CH | Et | Me | H | |
| 50 | NMeCH₂cPr | Et | Me | H | |
| 51 | NMe(CH₂)₂OH | Et | Me | H | |
| 52 | NMe(CH₂)₂OMe | Et | Me | H | |
| 53 | NMe(CH₂)₂OEt | Et | Me | H | |
| 54 | NMeCHMeCH₂OMe | Et | Me | H | |
| 55 | NMeCHMeCH₂OEt | Et | Me | H | |
| 56 | NHMe | Me | Et | H | |
| 57 | NHEt | Me | Et | H | |
| 58 | NH(n-Pr) | Me | Et | H | |
| 59 | NH(n-Bu) | Me | Et | H | |
| 60 | NHCH₂CH=CH₂ | Me | Et | H | |
| 61 | NHCH₂C≡CH | Me | Et | H | |
| 62 | NHCH₂cPr | Me | Et | H | |
| 63 | NH(CH₂)₂OH | Me | Et | H | |
| 64 | NH(CH₂)₂OMe | Me | Et | H | 0.52 (m,2H), 0.77 (m,2H), 1.00 (m,1H), 1.40 (t,3H), 2.31 (s,3H), 3.18 (s,3H), 3.40 (m,5H), 3.61 (m,2H), 3.97 (q,2H), 7.00 (d,1 H), 7.83 (d, 1H) |
| 65 | NH(CH₂)₂OEt | Me | Et | H | |
| 66 | NHCHMeCH₂OMe | Me | Et | H | |
| 67 | NHCHMeCH₂OEt | Me | Et | H | |
| 68 | NH(CH₂)₃OMe | Me | Et | H | |
| 69 | 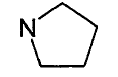 | Me | Et | H | |
| 70 | 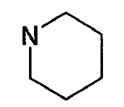 | Me | Et | H | |
| 71 | NMe₂ | Me | Et | H | |
| 72 | NMeEt | Me | Et | H | |
| 73 | NMe(n-Pr) | Me | Et | H | |
| 74 | NMe(n-Bu) | Me | Et | H | |
| 75 | NMeCH₂CH=CH₂ | Me | Et | H | |
| 76 | NMeCH₂C≡CH | Me | Et | H | |
| 77 | NMeCH₂cPr | Me | Et | H | |
| 78 | NMe(CH₂)₂OH | Me | Et | H | |
| 79 | NMe(CH₂)₂OMe | Me | Et | H | |
| 80 | NMe(CH₂)₂OEt | Me | Et | H | |
| 81 | NMeCHMeCH₂OMe | Me | Et | H | |
| 82 | NHCHMeCH₂OEt | Me | Et | H | |
| 83 | NHMe | Et | Et | H | |
| 84 | NHEt | Et | Et | H | |
| 85 | NH(n-Pr) | Et | Et | H | |
| 86 | NH(n-Bu) | Et | Et | H | |
| 87 | NHCH₂CH=CH₂ | Et | Et | H | |
| 88 | NHCH₂C≡CH | Et | Et | H | |
| 89 | NHCH₂cPr | Et | Et | H | |
| 90 | NH(CH₂)₂OH | Et | Et | H | |
| 91 | NH(CH₂)₂OMe | Et | Et | H | |
| 92 | NH(CH₂)₂OEt | Et | Et | H | |
| 93 | NHCHMeCH₂OMe | Et | Et | H | |
| 94 | NHCHMeCH₂OEt | Et | Et | H | |
| 95 | NH(CH₂)₃OMe | Et | Et | H | |
| 96 | 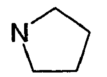 | Et | Et | H | |
| 97 | 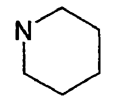 | Et | Et | H | |
| 98 | NMe₂ | Et | Et | H | |
| 99 | NMeEt | Et | Et | H | |
| 100 | NMe(n-Pr) | Et | Et | H | |
| 101 | NMe(n-Bu) | Et | Et | H | |
| 102 | NMeCH₂CH=CH₂ | Et | Et | H | |
| 103 | NMeCH₂C≡CH | Et | Et | H | |
| 104 | NMeCH₂cPr | Et | Et | H | |
| 105 | NMe(CH₂)₂OH | Et | Et | H | |
| 106 | NMe(CH₂)₂OMe | Et | Et | H | |
| 107 | NMe(CH₂)₂OEt | Et | Et | H | |
| 108 | NMeCHMeCH₂OMe | Et | Et | H | |
| 109 | NMeCHMeCH₂OEt | Et | Et | H | |
| 110 | NMe(CH₂)₃OMe | Me | Me | SO₂n-Pr | |
| 111 | NHEt | Me | Me | SO₂n-Pr | |
| 112 | NH(n-Pr) | Me | Me | SO₂n-Pr | |
| 113 | NH(n-Bu) | Me | Me | SO₂n-Pr | |
| 114 | NHCH₂CH=CH₂ | Me | Me | SO₂n-Pr | |
| 115 | NHCH₂C≡CH | Me | Me | SO₂n-Pr | |
| 116 | NHCH₂cPr | Me | Me | SO₂n-Pr | |
| 117 | NH(CH₂)₂OH | Me | Me | SO₂n-Pr | |
| 118 | NH(CH₂)₂OMe | Me | Me | SO₂n-Pr | 0.65 (m,2H), 0.85 (m,2H), 1.1 (t,3H), 1.58 (m,1H), 1.95 (m,2H), 2.3 (s,3H), 3.18 (s,3H), 3.3 (m,2H), 3.4 (m,2H), 3.4 (s,3H), 3.6 (m,2H), 3.78 (s,3H), 7.0 (d,1 H), 7.8 (d,1 H) |
| 119 | NH(CH₂)₂OEt | Me | Me | SO₂n-Pr | |
| 120 | NHCHMeCH₂OMe | Me | Me | SO₂n-Pr | |
| 121 | NHCHMeCH₂OEt | Me | Me | SO₂n-Pr | |
| 122 | NH(CH₂)₃OMe | Me | Me | SO₂n-Pr | |
| 123 | 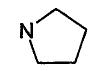 | Me | Me | SO₂n-Pr | |
| 124 | 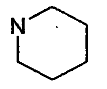 | Me | Me | SO₂n-Pr | |
| 125 | NMe₂ | Me | Me | SO₂n-Pr | |
| 126 | NMeEt | Me | Me | SO₂n-Pr | |
| 127 | NMe(n-Pr) | Me | Me | SO₂n-Pr | |
| 128 | NMe(n-Bu) | Me | Me | SO₂n-Pr | |
| 129 | NMeCH₂CH=CH₂ | Me | Me | SO₂n-Pr | |
| 130 | NMeCH₂C≡CH | Me | Me | SO₂n-Pr | |
| 131 | NMeCH₂cPr | Me | Me | SO₂n-Pr | |
| 132 | NMe(CH₂)₂OH | Me | Me | SO₂n-Pr | |
| 133 | NMe(CH₂)₂OMe | Me | Me | SO₂n-Pr | |
| 134 | NMe(CH₂)₂OEt | Me | Me | SO₂n-Pr | |
| 135 | NMeCHMeCH₂OMe | Me | Me | SO₂n-Pr | |
| 136 | NMe(CH₂)₃OMe | Me | Me | SO₂n-Pr | |
| 137 | NHMe | Me | Me | SO₂-(4-Me-Ph) | |
| 138 | NHEt | Me | Me | SO₂-(4-Me-Ph) | |
| 139 | NH(n-Pr) | Me | Me | SO₂-(4-Me-Ph) | |
| 140 | NH(n-Bu) | Me | Me | SO₂-(4-Me-Ph) | |
| 141 | NHCH₂CH=CH₂ | Me | Me | SO₂-(4-Me-Ph) | |
| 142 | NHCH₂C=CH | Me | Me | SO₂-(4-Me-Ph) | |
| 143 | NHCH₂cPr | Me | Me | SO₂-(4-Me-Ph) | |
| 144 | NH(CH₂)₂OH | Me | Me | SO₂-(4-Me-Ph) | |
| 145 | NH(CH₂)₂OMe | Me | Me | SO₂-(4-Me-Ph) | 0.82 (m,2H), 0.9 (m,2H), 2.08 (m,1 H), 2.32 (s,3H), 2.45 (s,3H), 3.18 (s,3H), 3.38 (t,2H), 3.4 (s,3H), 3.6 (t,2H), 3.6 (s,3H), 6.9 (d,1H), 7.35 (d,2H), 7.5 (d,2H), 7.65 (d, 1H) |
| 146 | N NH(CH₂)₂OEt | Me | Me | SO₂-(4-Me-Ph) | |
| 147 | NHCHMeCH₂OMe | Me | Me | SO₂-(4-Me-Ph) | |
| 148 | NHCHMeCH₂OEt | Me | Me | SO₂-(4-Me-Ph) | |
| 149 | NH(CH₂)₃OMe | Me | Me | SO₂-(4-Me-Ph) | |
| 150 | 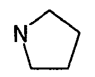 | Me | Me | SO₂-(4-Me-Ph) | |
| 151 | 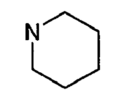 | Me | Me | SO₂-(4-Me-Ph) | |
| 152 | NMe₂ | Me | Me | SO₂-(4-Me-Ph) | |
| 153 | NMeEt | Me | Me | SO₂-(4-Me-Ph) | |
| 154 | NMe(n-Pr) | Me | Me | SO₂-(4-Me-Ph) | |
| 155 | NMe(n-Bu) | Me | Me | SO₂-(4-Me-Ph) | |
| 156 | NMeCH₂CH=CH₂ | Me | Me | SO₂-(4-Me-Ph) | |
| 157 | NMeCH₂C≡CH | Me | Me | SO₂-(4-Me-Ph) | |
| 158 | NMeCH₂cPr | Me | Me | SO₂-(4-Me-Ph) | |
| 159 | NMe(CH₂)₂OH | Me | Me | SO₂-(4-Me-Ph) | |
| 160 | NMe(CH₂)₂OMe | Me | Me | SO₂-(4-Me-Ph) | |
| 161 | NMe(CH₂)₂OEt | Me | Me | SO₂-(4-Me-Ph) | |
| 162 | NMeCHMeCH₂OMe | Me | Me | SO₂-(4-Me-Ph) | |
| 163 | NMe(CH₂)₃OMe | Me | Me | SO₂-(4-Me-Ph) | |
| 164 | NHMe | Me | Me | CH₂-(2,6-F₂-Ph) | |
| 165 | NHEt | Me | Me | CH₂-(2,6-F₂-Ph) | |
| 166 | NH(n-Pr) | Me | Me | CH₂-(2,6-F₂-Ph) | |
| 167 | NH(n-Bu) | Me | Me | CH₂-(2,6-F₂-Ph) | |
| 168 | NHCH₂CH=CH₂ | Me | Me | CH₂-(2,6-F₂-Ph) | |
| 169 | NHCH₂C≡CH | Me | Me | CH₂-(2,6-F₂-Ph) | |
| 170 | NHCH₂cPr | Me | Me | CH₂-(2,6-F₂-Ph) | |
| 171 | NH(CH₂)₂OH | Me | Me | CH₂-(2,6-F₂-Ph) | |
| 172 | NH(CH₂)₂OMe | Me | Me | CH₂-(2,6-F₂-Ph) | 0.58 (m,2H), 0.78 (m,2H), 1.42 (m,1H), 2.32 (s,3H), 3.18 (s,3H), 3.4 (t,2H), 3.4 (s,3H), 3.42 (s,2H), 3.6 (t,2H), 6.92 (m,2H), 7.02 (d,1H), 7.35 (m,1H), 7.8 (d,1H) |
| 173 | NH(CH₂)₂OEt | Me | Me | CH₂-(2,6-F₂-Ph) | |
| 174 | NHCHMeCH₂OMe | Me | Me | CH₂-(2,6-F₂-Ph) | |
| 175 | NHCHMeCH₂OEt | Me | Me | CH₂-(2,6-F₂-Ph) | |
| 176 | NH(CH₂)₃OMe | Me | Me | CH₂-(2,6-F₂-Ph) | |
| 177 | 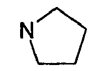 | Me | Me | CH₂-(2,6-F₂-Ph) | |
| 178 | 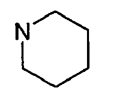 | Me | Me | CH₂-(2,6-F₂-Ph) | |
| 179 | NMe₂ | Me | Me | CH₂-(2,6-F₂-Ph) | |
| 180 | NMeEt | Me | Me | CH₂-(2,6-F₂-Ph) | |
| 181 | NMe(n-Pr) | Me | Me | CH₂-(2,6-F₂-Ph) | |
| 182 | NMe(n-Bu) | Me | Me | CH₂-(2,6-F₂-Ph) | |
| 183 | NMeCH₂CH=CH₂ | Me | Me | CH₂-(2,6-F₂-Ph) | |
| 184 | NMeCH₂C≡CH | Me | Me | CH₂-(2,6-F₂-Ph) | |
| 185 | NMeCH₂cPr | Me | Me | CH₂-(2,6-F₂-Ph) | |
| 186 | NMe(CH₂)₂OH | Me | Me | CH₂-(2,6-F₂-Ph) | |
| 187 | NMe(CH₂)₂OMe | Me | Me | CH₂-(2,6-F₂-Ph) | |
| 188 | NMe(CH₂)₂OEt | Me | Me | CH₂-(2,6-F₂-Ph) | |
| 189 | NMeCHMeCH₂OMe | Me | Me | CH₂-(2,6-F₂-Ph) | |
| 190 | NMe(CH₂)₂OMe | Me | Me | CH₂-(2,6-F₂-Ph) | |
| 191 | NH(c-Pr) | Me | Me | H | 0.48-0.64 (m,4H), 0.74-0.82 (m,4H), 1.05 (m,1 H), 2.48 (s,3H), 2.83 (m,1 H), 2.98 (s,3H), 3.61 (s,3H), 6.89 (d,1H), 7.76 (d,1H) |
| 192 | NH(i-Pr) | Me | Me | H | 0.53 (m,2H), 0.78 (m,2H), 1.02 (m,1H), 1.22 (d,6H), 2.27 (s,3H), 3.10 (s,3H), 3.60 (s,3H), 3.77 (m,1H), 6.96 (d,1 H), 7.82 (d, 1 H) |
| 193 | NH(i-Bu) | Me | Me | H | 0.54 (m,2H), 0.78 (m,2H), 1.01 (m,1 H), 1.04 (d,6H), 1.94 (m,1 H), 2.30 (s,3H), 3.02 (d,2H), 3.07 (s,3H), 3.61 (s,3H), 6.97 (d,1H), 7.80 (d,1H) |
| 194 | NH(s-Bu) | Me | Me | H | 0.54 (m,2H), 0.78 (m,2H), 0.95-1.06 (m,4H), 1.15 (d,3H), 1.49 (m,1H), 1.64 (m,1H), 2.27 (s,3H), 3.08 (s,3H), 3.56 (m,1 H), 3.61 (s,3H), 6.94 (d,1 H), 7.79 (d,1H) |
| 195 | NH(c-Pentyl) | Me | Me | H | 0.53 (m,2H), 0.77 (m,2H), 1.03 (m,1H), 1.46-1.67 (m,4H), 1.69-1.85 (m,2H), 1.88-2.01 (m,2H), 2.32 (s,3H), 3.07 (s,3H), 3.60 (s,3H), 3.96 (m,1H), 6.90 (d,1 H), 7.78 (d,1 H) |
| 196 | NH-Bzl | Me | Me | H | 0.54 (m,2H), 0.78 (m,2H), 0.98 (m,1H), 2.40 (s,3H), 2.70 (s,3H), 3.61 (s,3H), 4.38 (s,2H), 7.03 (d,1H), 7.24-7.38 (m,5H), 7.80 (d,1 H) |
| 197 | NH(CH₂)₂OAc | Me | Me | H | 0.54 (m,2H), 0.78 (m,2H), 0.98 (m,1H), 2.12 (s,3H), 2.32 (s,3H), 3.14 (s,3H), 3.47 (m,2H), 3.61 (s,3H), 4.32 (m,2H), 7.06 (d, 1H), 7.84 (d,1H) |
| 198 | NH(CH₂)₂O(i-Pr) | Me | Me | H | 0.54 (m,2H), 0.77 (m,2H), 1.00 (m,1H), 1.22 (d,6H), 2.30 (s,3H), 3.18 (s,3H), 3.37 (m,2H), 3.60 (s,3H), 3.62-3.73 (m,3H), 6.99 (d,1 H), 7.83 (d,1 H) |
| 199 | NHCH(Me)(c-Pr) | Me | Me | H | 0.10-0.24 (m,2H), 0.38-0.59 (m,4H), 0.77 (m,2H), 0.91-1.05 (m,2H), 1.24 (d,3H), 2.23 (s,3H), 3.00 (m,1H), 3.16 (s,3H), 3.60 (s,3H), 6.97 (d,1 H), 7.81 (d,1H) |
| 200 | NHCH(Et)(c-Pr) | Me | Me | H | 0.02 (m,1H), 0.21 (m,1H), 0.34 (m,1H), 0.48-0.60 (m,3H), 0.78 (m,2H), 0.91 (m,1 H), 0.98-1.11 (m,4H), 1.69 (m,2H), 2.22 (s,3H), 2.75 (m,1H), 3.16 (s,3H), 3.62 (s,3H), 6.95 (d, 1 H), 7.80 (d, 1H) |
| 201 | 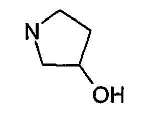 | Me | Me | H | 0.52 (m,2H), 0.78 (m,2H), 0.91 (m,1 H), 2.03-2.15 (m,1 H), 2.18-2.35 (m,1H), 2.32 (s,3H), 3.28 (s,3H), 3.31-3.42 (m,2H), 3.46-3.58 (m,2H), 3.61 (s,3H), 4.58 (m,1H), 7.36 (d,1H), 8.02 (d,1 H) |
| 202 | 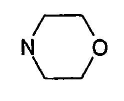 | Me | Me | H | 0.49 (m,2H), 0.78 (m,2H), 0.89 (m,1 H), 2.46 (s,3H), 2.97 (m,2H), 3.35 (s,3H), 3.55-3.66 (m,2H), 3.62 (s,3H), 3.80-3.97 (m,4H), 7.36 (d, 1H), 8.06 (d, 1H) |
| 203 | 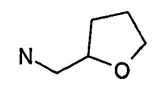 | Me | Me | H | 0.54 (m,2H), 0.77 (m,2H), 0.99 (m, 1 H), 1.59-1.73 (m,1 H), 1.88-2.12 (m,3H), 2.30 (s,3H), 3.18 (m,1H), 3.22 (s,3H), 3.41 (m,1H), 3.60 (s,3H), 3.74-3.84 (m,1H), 3.87-3.96 (m,1H), 4.05 (m,1 H), 6.98 (d,1 H), 7.83 (d,1 H) |
| 204 | 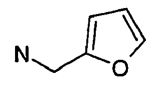 | Me | Me | H | 0.53 (m,2H), 0.78 (m,2H), 1.01 (m,1H), 2.38 (s,3H), 2.86 (s,3H), 3.61 (s,3H), 4.39 (s,2H), 6.23 (m,1H), 6.31 (m,1H), 7.03 (d,1 H), 7.33 (m, 1 H), 7.82 (d,1H) |
| 205 | 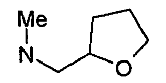 | Me | Me | H | |
| 206 | 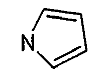 | Me | Me | H | 0.58 (m,2H), 0.82 (m,2H), 0.97 (m,1H), 1.99 (s,3H), 2.62 (s,3H), 3.61 (s,3H), 6.41 (m,2H), 6.78 (m,2H), 7.58 (d,1H), 8.18 (d,1H) |
| 207 | 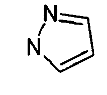 | Me | Me | H | 0.60 (b,2H), 0.82 (b,2H), 0.99 (m,1H), 1.95 (s,3H), 2.88 (s,3H), 3.61 (s,3H), 6.57 (m,1H), 7.67 (d,1H), 7.71 (d,1H), 7.82 (d,1 H), 8.17 (d,1 H) |
| 208 | 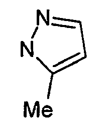 | Me | Me | H | 0.44-0.90 (m,4H), 1.03 (m,1H), 1.98 (s,3H), 2.41 (s,3H), 2.89 (s,3H), 3.62 (s,3H), 6.38 (d,1H), 7.62 (d,1H), 7.67 (d,1 H), 8.17 (d,1 H) |
| 209 | 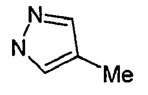 | Me | Me | H | 0.60 (b,2H), 0.82 (b,2H), 1.00 (m,1H), 1.96 (s,3H), 2.22 (s,3H), 2.89 (s,3H), 3.62 (s,3H), 7.48 (s,1H), 7.64 (s,1H), 7.66 (d,1H), 8.16 (d,1H) |
| 210 | 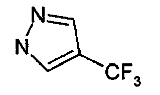 | Me | Me | H | 0.50-0.69 (m,2H), 0.84 (m,2H), 0.96 (m,1 H), 1.98 (s,3H), 2.97 (s,3H), 3.63 (s,3H), 7.71 (d,1 H), 7.98 (s,1H), 8.01 (s,1 H), 8.19 (d,1 H) |
| 211 | 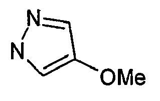 | Me | Me | H | 0.59 (b,2H), 0.82 (b,2H), 0.97 (m,1 H), 1.98 (s,3H), 2.93 (s,3H), 3.62 (s,3H), 3.83 (s,3H), 7.36 (s,1H), 7.57 (s,1H), 7.64 (d,1 H), 8.16 (d,1 H) |
| 212 | 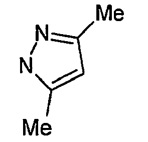 | Me | Me | H | 0.48-0.81 (m,3H), 0.88 (m,1H), 1.04 (m,1H), 1.92 (s,3H), 2.08 (s,3H), 2.35 (s,3H), 3.08 (s,3H), 3.62 (s,3H), 6.11 (s,1 H), 7.70 (d,1 H), 8.18 (d,1H) |
| 213 | 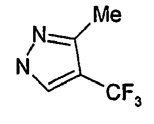 | Me | Me | H | 0.56 (b,1 H), 0.64 (b,1H), 0.84 (b,2H), 0.96 (m,1 H), 2.01 (s,3H), 2.44 (s,3H), 2.98 (s,3H), 3.61 (s,3H), 7.68 (d,1H), 7.86 (s, 1 H), 8.16 (d,1 H) |

### B. Formulierungsbeispiele

### 1. Stäubemittel

Ein Stäubemittel wird erhalten, indem man 10 Gew.-Teile einer Verbindung der allgemeinen Formel (I) und 90 Gew.-Teile Talkum als Inertstoff mischt und in einer Schlagmühle zerkleinert.

### 2. Dispergierbares Pulver

Ein in Wasser leicht dispergierbares, benetzbares Pulver wird erhalten, indem man 25 Gewichtsteile einer Verbindung der allgemeinen Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

### 3. Dispersionskonzentrat

Ein in Wasser leicht dispergierbares Dispersionskonzentrat wird erhalten, indem man 20 Gewichtsteile einer Verbindung der allgemeinen Formel (I), 6 Gew.-Teile Alkylphenolpolyglykolether (®Triton X 207), 3 Gew.-Teile Isotridecanolpolyglykolether (8 EO) und 71 Gew.-Teile paraffinischem Mineralöl (Siedebereich z.B. ca. 255 bis über 277°C) mischt und in einer Reibkugelmühle auf eine Feinheit von unter 5 Mikron vermahlt.

### 4. Emulgierbares Konzentrat

Ein emulgierbares Konzentrat wird erhalten aus 15 Gew.-Teilen einer Verbindung der allgemeinen Formel (I), 75 Gew.Teilen Cyclohexanon als Lösemittel und 10 Gew.-Teilen oxethyliertes Nonylphenol als Emulgator.

### 5. Wasserdispergierbares Granulat

Ein in Wasser dispergierbares Granulat wird erhalten, indem man

| | |
|---|---|
| 75 Gew.-Teile einer | Verbindung der allgemeinen Formel (I), |
| 10 " | ligninsulfonsaures Calcium, |
| 5 " | Natriumlaurylsulfat, |
| 3 " | Polyvinylalkohol und |
| 7 " | Kaolin |

mischt, auf einer Stiftmühle mahlt und das Pulver in einem Wirbelbett durch Aufsprühen von Wasser als Granulierflüssigkeit granuliert.

Ein in Wasser dispergierbares Granulat wird auch erhalten, indem man

| | |
|---|---|
| 25 Gew.-Teile einer | Verbindung der allgemeinen Formel (I), |
| 5 " | 2,2'-dinaphthylmethan-6,6'-disulfonsaures Natrium, |
| 2 " | oleoylmethyltaurinsaures Natrium, |
| 1 " | Polyvinylalkohol, |
| 17 " | Calciumcarbonat und |
| 50 " | Wasser |

auf einer Kolloidmühle homogenesiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Wirkung gegen Schadpflanzen beziehungsweise Schädigung von Kulturpflanzen im Vorauflauf

Samen von mono- und dikotylen Schadpflanzen sowie Samen von Mais, Sojabohne und Weizen werden in Papptöpfen in sandiger Lehmerde ausgelegt und mit Erde abgedeckt. Die in Form von benetzbaren Pulvern oder Emulsionskonzentraten formulierten erfindungsgemäßen und die aus dem Stand der Technik bekannten Verbindungen werden dann als wäßrige Suspension bzw. Emulsion mit einer Wasseraufwandmenge von umgerechnet 600 bis 800 I/ha in einer in den Tabellen 1 bis 10 angegebenen Dosierung auf die Oberfläche der Abdeckerde appliziert. Nach der Behandlung werden die Töpfe im Gewächshaus aufgestellt und unter guten Wachstumsbedingungen für die Schad- und Kulturpflanzen gehalten. Die optische Bonitur der Pflanzen- bzw. Auflaufschäden erfolgt nach dem Auflaufen der Pflanzen nach einer Versuchszeit von 3 bis 4 Wochen. Dabei wird festgestellt, daß die erfindungsgemäßen Verbindungen bei gleicher oder besserer herbizider Wirkung eine geringere Schädigung an Kulturpflanzen bewirken, als die aus dem Stand der Technik bekannten Verbindungen (Vergleichstabellen 1 bis 4, 6 bis 10).

### 2. Wirkung gegen Schadpflanzen beziehungsweise Schädigung von Kulturpflanzen im Nachauflauf

Samen von mono- und dikotylen Schadpflanzen sowie Samen von Mais, Sojabohne und Weizen werden in Papptöpfen in sandigem Lehmboden ausgelegt, mit Erde abgedeckt und im Gewächshaus unter guten Wachstumsbedingungen angezogen. Zwei bis drei Wochen nach der Aussaat werden die Schad- und Kulturpflanzen im Dreiblattstudium behandelt. Die als Spritzpulver bzw. als Emulsionskonzentrate formulierten erfindungsgemäßen und die aus dem Stand der Technik bekannten Verbindungen werden mit einer Wasseraufwandmenge von umgerechnet 600 bis 800 I/ha in einer in den Tabellen 1 bis 10 angegebenen Dosierung auf die Oberfläche der grünen Pflanzenteile gesprüht. Nach 3 bis 4 Wochen Standzeit der Versuchspflanzen im Gewächshaus unter optimalen Wachstumsbedingungen wird die Wirkung der Verbindungen im Vergleich zu Verbindungen, die im Stand der Technik offenbart sind, bonitiert. Dabei wird festgestellt, daß die erfindungsgemäßen Verbindungen bei gleicher oder besserer herbizider Wirkung eine geringere Schädigung an Kulturpflanzen bewirken, als die aus dem Stand der Technik bekannten Verbindungen (Vergleichstabellen 1 bis 4, 6 bis 10).

### 3. Wirkung gegen Schadpflanzen beziehungsweise Schädigung von Reis

Reispflanzen und in Reiskulturen typische Schadpflanzen werden im Gewächshaus unter Paddyreis-Bedingungen (Anstauhöhe des Wassers: 2-3 cm) angezogen. Nach der Behandlung mit den erfindungsgemäßen und den aus dem Stand der Technik bekannten Verbindungen werden die Versuchspflanzen im Gewächshaus unter optimalen Wachstumsbedingungen aufgestellt und während der gesamten Versuchszeit so gehalten. Etwa drei Wochen nach der Applikation erfolgt die Auswertung mittels optischer Bonitur der Pflanzenschäden. Dabei wird festgestellt, daß die erfindungsgemäßen Verbindungen bei gleicher oder besserer herbizider Wirkung eine geringere Schädigung an Reispflanzen bewirken, als die aus dem Stand der Technik bekannten Verbindungen (Vergleichstabellen 2 und 5).

**Tabelle B: Erfindungsgemäße Verbindungen**

| **Struktur** | **Struktur** |
|---|---|
| 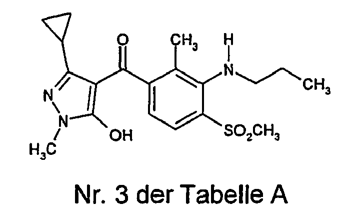 | 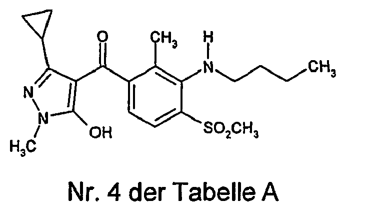 |
| 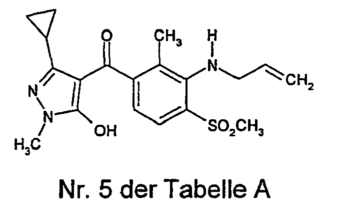 | 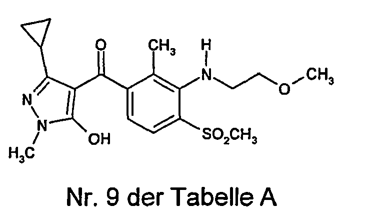 |
| 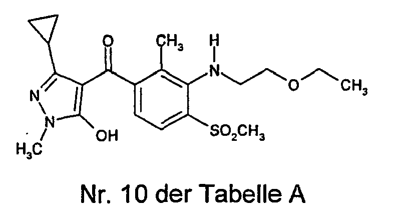 | 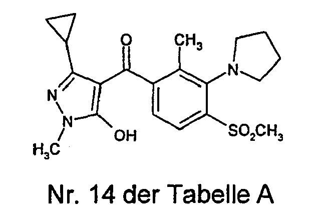 |
| 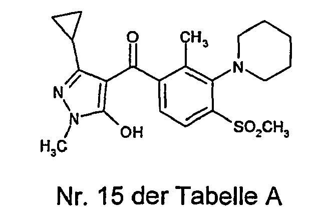 | |

**Tabelle C: Aus dem Stand der Technik bekannte Verbindungen**

| **Struktur** | **Struktur** |
|---|---|
| 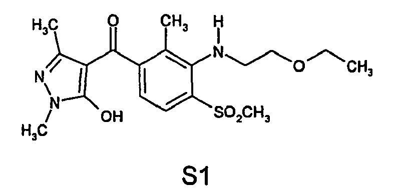 | 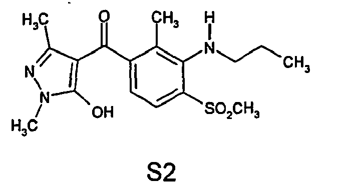 |
| 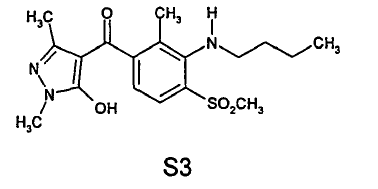 | 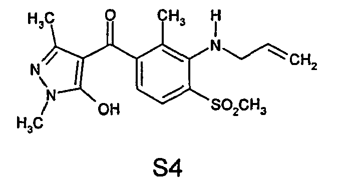 |
| 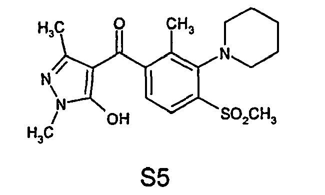 | 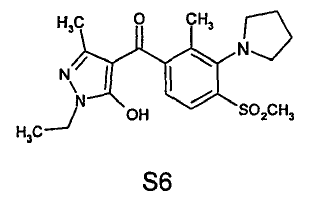 |
| 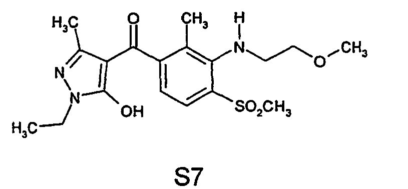 | |

Die in folgenden Vergleichstabellen verwendeten Abkürzungen bedeuten:

| (Schadpflanzen) | | | |
|---|---|---|---|
| AVEFA | Avena fatua | DIGSA | Digitaria sanguinalis |
| ECHCG | Echinochloa crus galli | GALAP | Galium aparine |
| MATIN | Matricaria inodora | PHBPU | Pharbitis purpureum |
| SETVI | Setaria viridis | STEME | Stellaria media |
| VERPE | Veronica persica | VIOTR | Viola tricolor |

| (Kulturpflanzen) | | | |
|---|---|---|---|
| GLXMA | Glycine max (Sojabohne) | ORYSA | Oryza sativa (Reis) |
| TRZAS | Triticum aestivum (Weizen) | ZEAMX | Zea mays (Mais) |

**Vergleichstabelle 1: Nachauflauf**

| Verbindung | Dosierung [g a. i/ha] | Herbizide Wirkung | | | | Schädigung der Kulturpflanzen |
|---|---|---|---|---|---|---|
| | | DIGSA | ECHCG | MATIN | VIOTR | ZEAMX |
| Nr. 3 | 80 | 90% | 90% | 70% | 90% | 30% |
| S2 | 80 | 80% | 80% | 30% | 80% | 60% |

**Vergleichstabelle 2: Nachauflauf**

| Verbindung | Dosierung [g a.i./ha] | Herbizide Wirkung | | Schädigung der Kulturpflanzen | |
|---|---|---|---|---|---|
| | | GALAP | VERPE | GLXMA | ORYSA |
| Nr.4 | 20 | 80% | 60% | 0% | 0% |
| S3 | 20 | 40% | 20% | 20% | 30% |

**Vergleichstabelle 3: Nachauflauf**

| Verbindung | Dosierung [g a.i./ha] | Herbizide Wirkung | | | Schädigung der Kulturpflanzen | |
|---|---|---|---|---|---|---|
| | | GALAP | MATIN | STEME | GLXMA | ZEAMX |
| Nr. 5 | 80 | 80% | 50% | 100% | 0% | 10% |
| S4 | 80 | 80% | 30% | 100% | 50% | 30% |

**Vergleichstabelle 4: Nachauflauf**

| Verbindung | Dosierung [g a.i./ha] | Herbizide Wirkung | | Schadigung der Kulturpflanzen |
|---|---|---|---|---|
| | | MATIN | STEME | ZEAMX |
| Nr. 15 | 80 | 60% | 70% | 0% |
| S5 | 80 | 40% | 20% | 10% |

**Vergleichstabelle 5: Nachauflauf**

| Verbindung | Dosierung [g a.i./ha] | Herbizide Wirkung | Schädigung der Kulturpflanzen |
|---|---|---|---|
| | | SETVI | ORYSA |
| Nr. 14 | 320 | 100% | 10% |
| S6 | 320 | 90% | 20% |

**Vergleichstabelle 6: Nachauflauf**

| Verbindung | Dosierung [g a.i./ha] | Herbizide Wirkung | | Schadigung der Kulturpflanzen | |
|---|---|---|---|---|---|
| | | ECHCG | MATIN | GLXMA | TRAZS |
| Nr. 14 | 320 | 80% | 50% | 10% | 0% |
| S6 | 320 | 50% | 0% | 20% | 10% |

**Vergleichstabelle 7: Vorauflauf**

| Verbindung | Dosierung [g a.i./ha] | Herbizide Wirkung | Schädigung der Kulturpflanzen |
|---|---|---|---|
| | | GALAP | ZEAMX |
| Nr. 9 | 320 | 80% | 0% |
| S7 | 320 | 70% | 20% |

**Vergleichstabelle 8: Nachauflauf**

| Verbindung | Dosierung [g a.i/ha] | Herbizide Wirkung | | Schädigung der Kulturpflanzen | |
|---|---|---|---|---|---|
| | | AVEFA | MATIN | GLXMA | ZEAMX |
| Nr. 9 | 320 | 70% | 80% | 20% | 0% |
| S7 | 320 | 50% | 70% | 60% | 20% |

**Vergleichstabelle 9: Nachauflauf**

| Verbindung | Dosierung [g a.i./ha] | Herbizide Wirkung | | | Schädigung der Kulturpflanzen |
|---|---|---|---|---|---|
| | | MATIN | PHBPU | VERPE | GLXMA |
| Nr. 10 | 320 | 100% | 100% | 100% | 20% |
| S1 | 320 | 60% | 90% | 100% | 90% |

**Vergleichstabelle 10: Nachauflauf**

| Verbindung | Dosierung [g a.i./ha] | Herbizide Wirkung | | | | Schädigung der Kulturpflanzen | |
|---|---|---|---|---|---|---|---|
| | | ECHCG | MATIN | PHBPU | VERPE | GLXMA | ZEAMX |
| Nr. 10 | 320 | 100% | 100% | 100% | 100% | 20% | 0% |
| S7 | 320 | 90% | 70% | 40% | 90% | 60% | 20% |

## Patentansprüche

1. Verbindungen der Formel (I) oder deren Salze 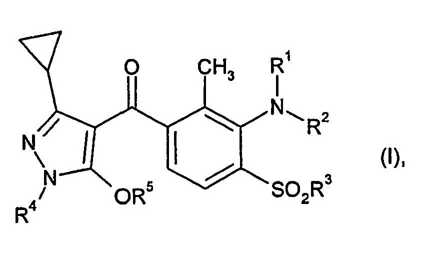 worin
R¹ und R² bedeuten unabhängig voneinander Wasserstoff, Furan-2-yl, Tetrahydrofuran-2-yl-methyl,
oder durch m Reste aus der Gruppe Fluor, Chlor, Brom, Cyano, Hydroxy, (C₁-C₄)-Alkyl, (C₁-C₄)-Alkoxy und (C₁-C₄)-Alkoxy-(C₁-C₄)-alkoxy substituiertes (C₁-C₄)-Alkyl, (C₃-C₄)-Alkenyl, (C₃-C₄)-Alkinyl, (C₃-C₆)-Cycloalkyl, (C₃-C₆)-Cycloalkenyl, (C₃-C₆)-Cycloalkyl-(C₁-C₄)-alkyl oder (C₃-C₆)-Cycloalkenyl-(C₁-C₄)-alkyl, wobei R¹ und R² nicht beide gleichzeitig Wasserstoff bedeuten,
oder
NR¹R² bedeutet einen 4- bis 7-gliedrigen gesättigten, teilweise gesättigten, vollständig ungesättigten oder aromatischen Ring enthaltend als Ringatome n Heteroatome aus der Gruppe Stickstoff, Sauerstoff und Schwefel, der durch m Reste aus der Gruppe Fluor, Chlor, Brom, Iod, Cyano, Hydroxy, Trifluormethyl, Nitro, (C₁-C₄)-Alkyl, (C₁-C₄)-Alkoxy, Fluor-(C₁-C₃)-alkyl, Fluor-(C₁-C₃)-alkoxy oder (C₁-C₃)-Alkoxymethyl substituiert ist;
R³ bedeutet Methyl, Ethyl oder iso-Propyl;
R⁴ bedeutet (C₁-C₄)-Alkyl, (C₃-C₄)-Alkenyl oder (C₃-C₄)-Alkinyl;
R⁵ bedeutet Wasserstoff, (C₁-C₄)-Alkylsulfonyl, (C₃-C₄)-Alkenylsulfonyl, (C₃-C₄)-Alkinylsulfonyl,
oder durch m Reste aus der Gruppe Fluor, Chlor, Brom, Cyano, Trifluormethyl, Nitro, Methyl, Ethyl, Methoxy und Ethoxy substituiertes Phenylsulfonyl oder Benzyl,
m bedeutet 0, 1, 2 oder 3;
n bedeutet 1, 2 oder 3.

2. Verbindungen nach Anspruch 1, worin
R¹ und R² bedeuten unabhängig voneinander Wasserstoff,
oder durch m Reste aus der Gruppe Fluor, Chlor, Brom, Cyano, Hydroxy, (C₁-C₄)-Alkyl, (C₁-C₄)-Alkoxy und (C₁-C₄)-Alkoxy-(C₁-C₄)-alkoxy substituiertes (C₁-C₄)-Alkyl, (C₃-C₄)-Alkenyl, (C₃-C₄)-Alkinyl, (C₃-C₆)-Cycloalkyl, (C₃-C₆)-Cycloalkenyl, (C₃-C₆)-Cycloalkyl-(C₁-C₄)-alkyl oder (C₃-C₆)-Cycloalkenyl-(C₁-C₄)-alkyl, wobei R¹ und R² nicht beide gleichzeitig Wasserstoff bedeuten,
oder
NR¹R² bedeutet einen 4- bis 7-gliedrigen gesättigten, teilweise gesättigten, vollständig ungesättigten oder aromatischen Ring enthaltend als Ringatome n Heteroatome aus der Gruppe Stickstoff, Sauerstoff und Schwefel, der durch m Reste aus der Gruppe Fluor, Chlor, Brom, Iod, Cyano, Trifluormethyl, Nitro, (C₁-C₄)-Alkyl, (C₁-C₄)-Alkoxy, Fluor-(C₁-C₃)-alkyl, Fluor-(C₁-C₃)-alkoxy oder (C₁-C₃)-Alkoxymethyl substituiert ist.

3. Verbindungen nach Anspruch 1 oder 2, worin
R¹ und R² unabhängig voneinander Wasserstoff, Methyl, Butyl, Ethyl, Propyl, Propenyl, Propinyl, Cyclopropyl, Cyclopropylmethyl, Methoxyethyl, Methoxypropyl, 2-Methoxy-1-methylethyl, 2-Ethoxy-1-methylethyl, Hydroxyethyl oder Ethoxyethyl bedeuten,
oder
NR¹R² bilden einen Rest aus der Gruppe 1-Pyrrolyl, 1-Pyrrolidinyl, 1-Pyrazolyl, 1-Piperidin, 1-Morpholinyl und 1-Piperazinyl, der durch m Reste aus der Gruppe Fluor, Chlor, Brom, Iod, Cyano, Trifluormethyl, (C₁-C₄)-Alkyl und (C₁-C₄)-Alkoxy substituiert ist.

4. Verbindungen nach einem der Ansprüche 1 bis 3, worin
R¹ und R² unabhängig voneinander Wasserstoff, Methyl, Butyl, Ethyl, Propyl, Propenyl, Propinyl, Cyclopropyl, Cyclopropylmethyl, Methoxyethyl, Methoxypropyl, 2-Methoxy-1-methylethyl, 2-Ethoxy-1-methylethyl, Hydroxyethyl oder Ethoxyethyl bedeuten,
oder
NR¹R² bilden einen Rest aus der Gruppe 1-Pyrrolyl, 1-Pyrrolidinyl, 1-Pyrazolyl, 1-Piperidin, 1-Morpholinyl und 1-Piperazinyl, und
R⁵ Wasserstoff, Propylsulfonyl, Tosyl oder 2,6-Difluorbenzyl bedeutet.

5. Verbindungen nach einem der Ansprüche 1 bis 4, worin
R³ Methyl oder Ethyl bedeutet;
R⁴ Methyl oder Ethyl bedeutet, und
R⁵ Wasserstoff bedeutet.

6. Herbizide Mittel, **gekennzeichnet durch** einen herbizid wirksamen Gehalt an mindestens einer Verbindung der allgemeinen Formel (I) gemäß einem der Ansprüche 1 bis 5.

7. Herbizide Mittel nach Anspruch 6 in Mischung mit Formulierungshilfsmitteln.

8. Verfahren zur Bekämpfung unerwünschter Pflanzen, **dadurch gekennzeichnet, dass** man eine wirksame Menge mindestens einer Verbindung der allgemeinen Formel (I) nach einem der Ansprüche 1 bis 5 oder eines herbiziden Mittels nach Anspruch 6 oder 7 auf die Pflanzen oder auf den Ort des unerwünschten Pflanzenwachstums appliziert.

9. Verwendung von Verbindungen der allgemeinen Formel (I) nach einem der Ansprüche 1 bis 5 oder von herbiziden Mitteln nach Anspruch 6 oder 7 zur Bekämpfung unerwünschter Pflanzen.

10. Verwendung nach Anspruch 9, worin die Verbindungen der allgemeinen Formel (I) zur Bekämpfung unerwünschter Pflanzen in Kulturen von Nutzpflanzen eingesetzt werden.

11. Verwendung nach Anspruch 9 oder 10, worin die Nutzpflanzen transgene Nutzpflanzen sind.

## Claims

1. A compound of the formula (I) or salt thereof 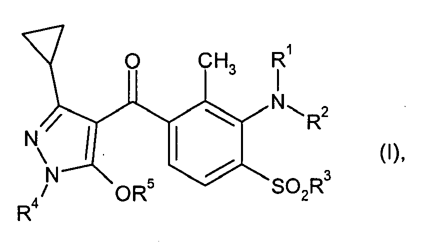 in which
R¹ and R² independently of one another are hydrogen, furan-2-yl, tetrahydrofuran-2-yl-methyl,
or (C₁-C₄)-alkyl, (C₃-C₄)-alkenyl, (C₃-C₄)-alkynyl, (C₃-C₆)-cycloalkyl, (C₃-C₆)-cycloalkenyl, (C₃-C₆)-cycloalkyl-(C₁-C₄)-alkyl or (C₃-C₆)-cycloalkenyl-(C₁-C₄)-alkyl substituted by m radicals from the group consisting of fluorine, chlorine, bromine, cyano, hydroxyl, (C₁-C₄)-alkyl, (C₁-C₄)-alkoxy and (C₁-C₄)-alkoxy-(C₁-C₄)-alkoxy, where R¹ and R² are not simultaneously hydrogen,
or
NR¹R² is a 4- to 7-membered saturated, partially saturated, fully unsaturated or aromatic ring comprising as ring atoms n heteroatoms from the group consisting of nitrogen, oxygen and sulfur which ring is substituted by m radicals from the group consisting of fluorine, chlorine, bromine, iodine, cyano, hydroxyl, trifluoromethyl, nitro, (C₁-C₄)-alkyl, (C₁-C₄)-alkoxy, fluoro-(C₁-C₃)-alkyl, fluoro-(C₁-C₃)-alkoxy or (C₁-C₃)-alkoxymethyl;
R³ is methyl, ethyl or isopropyl;
R⁴ is (C₁-C₄)-alkyl, (C₃-C₄)-alkenyl or (C₃-C₄)-alkynyl;
R⁵ is hydrogen, (C₁-C₄)-alkylsulfonyl, (C₃-C₄)-alkenylsulfonyl, (C₃-C₄)-alkynylsulfonyl,
or phenylsulfonyl or benzyl substituted by m radicals from the group consisting of fluorine, chlorine, bromine, cyano, trifluoromethyl, nitro, methyl, ethyl, methoxy and ethoxy,
m is 0, 1, 2 or 3;
n is 1, 2 or 3.

2. The compound as claimed in claim 1 in which
R¹ and R² independently of one another are hydrogen, or (C₁-C₄)-alkyl, (C₃-C₄)-alkenyl, (C₃-C₄)-alkynyl, (C₃-C₆)-cycloalkyl, (C₃-C₆)-cycloalkenyl, (C₃-C₆)-cycloalkyl-(C₁-C₄)-alkyl or (C₃-C₆)-cycloalkenyl-(C₁-C₄)-alkyl substituted by m radicals from the group consisting of fluorine, chlorine, bromine, cyano, hydroxyl, (C₁-C₄)-alkyl, (C₁-C₄)-alkoxy and (C₁-C₄)-alkoxy-(C₁-C₄)-alkoxy, where R¹ and R² are not simultaneously hydrogen,
or
NR¹R² is a 4- to 7-membered saturated, partially saturated, fully unsaturated or aromatic ring comprising as ring atoms n heteroatoms from the group consisting of nitrogen, oxygen and sulfur which ring is substituted by m radicals from the group consisting of fluorine, chlorine, bromine, iodine, cyano, trifluoromethyl, nitro, (C₁-C₄)-alkyl, (C₁-C₄)-alkoxy, fluoro-(C₁-C₃)-alkyl, fluoro-(C₁-C₃)-alkoxy or (C₁-C₃)-alkoxymethyl.

3. The compound as claimed in claim 1 or 2 in which
R¹ and R² independently of one another are hydrogen, methyl, butyl, ethyl, propyl, propenyl, propynyl, cyclopropyl, cyclopropylmethyl, methoxyethyl, methoxypropyl, 2-methoxy-1-methylethyl, 2-ethoxy-1-methylethyl, hydroxyethyl or ethoxyethyl,
or
NR¹R² form a radical from the group consisting of 1-pyrrolyl, 1-pyrrolidinyl, 1-pyrazolyl, 1-piperidine, 1-morpholinyl and 1-piperazinyl which is substituted by m radicals from the group consisting of fluorine, chlorine, bromine, iodine, cyano, trifluoromethyl, (C₁-C₄)-alkyl and (C₁-C₄)-alkoxy.

4. The compound as claimed in any of claims 1 to 3 in which
R¹ and R² independently of one another are hydrogen, methyl, butyl, ethyl, propyl, propenyl, propynyl, cyclopropyl, cyclopropylmethyl, methoxyethyl, methoxypropyl, 2-methoxy-1-methylethyl, 2-ethoxy-1-methylethyl, hydroxyethyl or ethoxyethyl,
or
NR¹R² form a radical from the group consisting of 1-pyrrolyl, 1-pyrrolidinyl, 1-pyrazolyl, 1-piperidine, 1-morpholinyl and 1-piperazinyl and
R⁵ is hydrogen, propylsulfonyl, tosyl or 2,6-difluorobenzyl.

5. The compound as claimed in any of claims 1 to 4 in which
R³ is methyl or ethyl;
R⁴ is methyl or ethyl, and
R⁵ is hydrogen.

6. A herbicidal composition comprising a herbicidally effective amount of at least one compound of the formula (I) as claimed in any of claims 1 to 5.

7. The herbicidal composition as claimed in claim 6 as a mixture with formulating assistants.

8. A method of controlling unwanted plants, which comprises applying to the plants or to the locus of unwanted plant growth an effective amount of at least one compound of the formula (I) as claimed in any of claims 1 to 5 or of a herbicidal composition as claimed in claim 6 or 7.

9. The use of a compound of the formula (I) as claimed in any of claims 1 to 5 or of a herbicidal composition as claimed in claim 6 or 7 for controlling unwanted plants.

10. The use as claimed in claim 9, wherein the compound of the formula (I) is used for controlling unwanted plants in crops of useful plants.

11. The use as claimed in claim 9 or 10, wherein the useful plants are transgenic useful plants.

## Revendications

1. Composés de formule (I) ou leurs sels 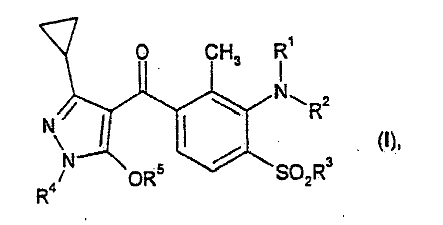 où
R¹ et R², indépendamment l'un de l'autre, représentent un atome d'hydrogène, un groupe furan-2-yle, tétrahydrofuran-2-yl-méthyle,
ou un groupe alkyle en C₁-C₄, alcényle en C₃-C₄, alcynyle en C₃-C₄, cycloalkyle en C₃-C₆, cycloalcényle en C₃-C₆, (cycloalkyl en C₃-C₆)-(alkyle en C₁-C₄) ou (cycloalcényl en C₃-C₆)-(alkyle en C₁-C₄) substitué par m radicaux pris dans le groupe constitué par un atome de fluor, de chlore, de brome, un groupe cyano, hydroxy, alkyle en C₁-C₄, alkoxy en C₁-C₄ et (alkoxy en C₁-C₄)-(alkoxy en C₁-C₄),
où R¹ et R² ne représentent pas simultanément un atome d'hydrogène,
ou
NR¹R² représente un cycle de 4 à 7 chaînons saturé, partiellement saturé, complètement insaturé ou aromatique contenant en tant que chaînons n hétéroatomes pris dans le groupe constitué par un atome d'azote, d'oxygène et de soufre, qui est substitué par m radicaux pris dans le groupe constitué par un atome de fluor, de chlore, de brome, d'iode, un groupe cyano, hydroxy, trifluorométhyle, nitro, alkyle en C₁-C₄, alkoxy en C₁-C₄, fluoroalkyle en C₁-C₃, fluoroalkoxy en C₁-C₃ ou alkoxyméthyle en C₁-C₃ ;
R³ représente un groupe méthyle, éthyle ou iso-propyle ;
R⁴ représente un groupe alkyle en C₁-C₄, alcényle en C₃-C₄ ou alcynyle en C₃-C₄ ;
R⁵ représente un atome d'hydrogène, un groupe (alkyl en C₁-C₄) sulfonyle, (alcényl en C₃-C₄) sulfonyle, (alcynyl en C₃-C₄) sulfonyle,
ou un groupe phénylsulfonyle ou benzyle substitué par m radicaux pris dans le groupe constitué par un atome de fluor, de chlore, de brome, un groupe cyano, trifluorométhyle, nitro, méthyle, éthyle, méthoxy et éthoxy ;
m vaut 0, 1, 2 ou 3 ;
n vaut 1, 2 ou 3.

2. Composés selon la revendication 1, où
R¹ et R², indépendamment l'un de l'autre, représentent un atome d'hydrogène,
ou un groupe alkyle en C₁-C₄, alcényle en C₃-C₄, alcynyle en C₃-C₄, cycloalkyle en C₃-C₆, cycloalcényle en C₃-C₆, (cycloalkyl en C₃-C₆) - (alkyle en C₁-C₄) ou (cycloalcényl en C₃-C₆) - (alkyle en C₁-C₄) substitué par m radicaux pris dans le groupe constitué par un atome de fluor, de chlore, de brome, un groupe cyano, hydroxy, alkyle en Ci-C₄, alkoxy en C₁-C₄ et (alkoxy en C₁-C₄) - (alkoxy en C₁-C₄), où R¹ et R² ne représentent pas simultanément un atome d'hydrogène,
ou
NR¹R² représente un cycle de 4 à 7 chaînons saturé, partiellement saturé, complètement insaturé ou aromatique contenant en tant que chaînons n hétéroatomes pris dans le groupe constitué par un atome d'azote, d'oxygène et de soufre, qui est substitué par m radicaux pris dans le groupe constitué par un atome de fluor, de chlore, de brome, d'iode, un groupe cyano, trifluorométhyle, nitro, alkyle en C₁-C₄, alkoxy en C₁-C₄, fluoroalkyle en C₁-C₃, fluoroalkoxy en C₁-C₃ ou alkoxyméthyle en C₁-C₃.

3. Composés selon la revendication 1 ou 2,
où
R¹ et R², indépendamment l'un de l'autre, représentent un atome d'hydrogène, un groupe méthyle, butyle, éthyle, propyle, propényle, propynyle, cyclopropyle, cyclopropylméthyle, méthoxyéthyle, méthoxypropyle, 2-méthoxy-1-méthyléthyle, 2-éthoxy-1-méthyléthyle, hydroxyéthyle ou éthoxyéthyle,
ou
NR¹R² forment un radical pris dans le groupe constitué par 1-pyrrolyle, 1-pyrrolidinyle, 1-pyrazolyle, 1-pipéridine, 1-morpholinyle et 1-pipérazinyle, qui est substitué par m radicaux pris dans le groupe constitué par un atome de fluor, de chlore, de brome, d'iode, un groupe cyano, trifluorométhyle, alkyle en C₁-C₄ et alkoxy en C₁-C₄.

4. Composés selon l'une des revendications 1 à 3, où
R¹ et R², indépendamment l'un de l'autre, représentent un atome d'hydrogène, un groupe méthyle, butyle, éthyle, propyle, propényle, propynyle, cyclopropyle, cyclopropylméthyle, méthoxyéthyle, méthoxypropyle, 2-méthoxy-1-méthyléthyle, 2-éthoxy-1-méthyléthyle, hydroxyéthyle ou éthoxyéthyle,
ou
NR¹R² forment un reste pris dans le groupe constitué par 1-pyrrolyle, 1-pyrrolidinyle, 1-pyrazolyle, 1-pipéridine, 1-morpholinyle et 1-pipérazinyle et
R⁵ représente un atome d'hydrogène, un groupe propylsulfonyle, tosyle ou 2,6-difluorobenzyle.

5. Composés selon l'une des revendications 1 à 4, où
R³ représente un groupe méthyle ou éthyle ;
R⁴ représente un groupe méthyle ou éthyle, et
R⁵ représente un atome d'hydrogène.

6. Agent herbicide, **caractérisé par** une teneur à efficacité herbicide en au moins un composé de formule générale (I) selon l'une des revendications 1 à 5.

7. Agent herbicide selon la revendication 6 en mélange avec des formulants.

8. Procédé pour la lutte contre les plantes indésirables, **caractérisé en ce qu'**une quantité efficace d'au moins un composé de formule générale (I) selon l'une des revendications 1 à 5 ou d'un agent herbicide selon la revendication 6 ou 7 est appliquée aux plantes ou au site de croissance des plantes indésirables.

9. Utilisation de composés de formule générale (I) selon l'une des revendications 1 à 5 ou d'agents herbicides selon la revendication 6 ou 7, pour la lutte contre les plantes indésirables.

10. Utilisation selon la revendication 9, où les composés de formule générale (I) sont utilisés pour la lutte contre les plantes indésirables dans les cultures de plantes utiles.

11. Utilisation selon la revendication 9 ou 10, où les plantes utiles sont les plantes utiles transgéniques.
